(19) Europäisches Patentamt
European Patent Office
Office européen des brevets

(11) EP 3 443 952 A1

(12) EUROPEAN PATENT APPLICATION
published in accordance with Art. 153(4) EPC

(43) Date of publication:
20.02.2019 Bulletin 2019/08

(21) Application number: 17781845.7

(22) Date of filing: 08.04.2017

(51) Int Cl.:
*A61K 9/127* (2006.01) *A61K 31/7088* (2006.01)
*A61K 48/00* (2006.01) *A61K 47/18* (2017.01)
*A61K 47/22* (2006.01) *A61K 47/24* (2006.01)
*A61K 47/28* (2006.01) *A61K 47/34* (2017.01)
*A61P 43/00* (2006.01)

(86) International application number:
PCT/CN2017/079810

(87) International publication number:
WO 2017/177869 (19.10.2017 Gazette 2017/42)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD

(30) Priority: 11.04.2016 CN 201610222630

(71) Applicant: Biomics Biotechnologies Co., Ltd.
Nantong, Jiangsu 226016 (CN)

(72) Inventors:
- CHEN, Jianxin
Nantong
Jiangsu 226016 (CN)
- PENG, Wei
Nantong
Jiangsu 226016 (CN)
- LI, Tiejun
Nantong
Jiangsu 226016 (CN)
- NI, Yan
Nantong
Jiangsu 226016 (CN)
- FENG, Wenjian
Nantong
Jiangsu 226016 (CN)

(74) Representative: Haseltine Lake LLP
Redcliff Quay
120 Redcliff Street
Bristol BS1 6HU (GB)

# (54) LIPOSOME PREPARATION HAVING HIGH-CONTENT CATIONIC LIPID COMPOUND AND USE THEREOF

(57) Provided is a liposome for use in the delivery of nucleic acids, comprising a cationic lipid compound, a phospholipid and a long-acting lipid, wherin the weight percentage content of the cationic lipid compound is from 50% to 90%, and the cationic lipid compound is composed of a lipid compound and a cholesterol lipid compound. Also provided are a nucleic acid-liposome preparation comprising the above-mentioned liposome and a nucleic acid encapsulated therein, and a preparation method for the preparation. The content of the nucleic acid drug loaded in the nucleic acid-liposome preparation is comparatively high, and hence, under the conditions of administration of the same dose, the intake amount of the cationic lipid compound relatively decreases, such that the toxicity is lowered.

EP 3 443 952 A1

## Description

### CROSS REFERENCE TO RELATED APPLICATIONS

This application claims the benefit of, and priority to, Chinese Patent Application Serial No. 201610222630.2, entitled "Liposome Formulation Comprising Cationic Lipid Compounds in High Amount and the Use Thereof', filed on April 11, 2016, the entire disclosures of which are incorporated by reference.

### FIELD OF THE INVENTION

The present invention relates to a liposome formulation, more particularly, to a liposome formulation comprising cationic lipid compounds in high amount and the use thereof.

### BACKGROUND OF THE INVENTION

In recent ten years, RNA interference (RNAi) is found to be a self protection mechanism having a function for modulating gene expression in organisms. RNAi is a specific gene silence phenomenon mediated by double-stranded RNA (dsRNA), in which specific enzymes involve. RNAi blocks gene expression at transcriptional level, post-transcriptional level and translational level. With the development of RNAi technology, a variety of nucleic acid medicaments designed and developed based on RNAi mechanism enter clinical trial stage. The nucleic acid medicaments for modulating abnormal gene expression will be a promised effective route for treating diseases.

In recent years, a series of nucleic acid medicaments based on RNAi mechanism show well development, which include small interfering RNAs (siRNAs), microRNAs (miRNAs), non-coding RNAs (ncRNAs), antisense RNAs/DNAs, small ligand RNAs (sliRNAs) and small active RNAs (saRNAs) and the like. These nucleic acids exhibit their functions via various gene modulation mechanisms including RNAi. For example, siRNAs and miRNAs modulate expression level of specific genes in cells via RNAi mechanism. After siRNAs or miRNAs go into cells, these double-stranded RNAs are able to combine with specific proteins in cells to form RNA inducing silencing complexes (RISCs). After unwinding of siRNAs in RISCs, they combine with mRNAs through sequence complementary identification and then mRNAs are cutted to achieve down-regulation of the gene expression of mRNAs. RNAi provides a gene therapy by complementing to the target genes and blocking the expression of mRNA encoding protein.

Vector systems based on the liposomes are used to deliver the nucleic acid medicaments to systematically administer siRNAs encapsulated within the cationic liposomes. There are reports about the effeciacy of the nucleic acid liposome formulations *in vivo* and *in vitro.* However, there are still several problems in application of nucleic acid liposome formulations: 1) lack of effective *in vivo* delivery systems; 2) low drug loading capacity for the current liposome formulations; and 3) high *in vivo* toxicity of the nucleic acid liposome formulations.

In order to overcome the above problems, there is a need to provide a liposome formulation comprising cationic lipid compounds in high amount, which can safely deliver nucleic acid medicaments in high amount into body.

### SUMMARY OF THE INVENTION

In one aspect, a liposome for delivery of nucleic acids is provided herein, comprising cationic lipid compounds, a phospholipid and a PEGylated lipid, in which, the cationic lipid compounds have an amount from 50wt% to 90wt%, the phospholipid has an amount from 5wt% to 10wt%, and the PEGylated lipid has an amount from 0wt% to 10wt%. The cationic lipid compounds consist of a lipid compound and a cholesterol lipid compound. The molar ratio between the lipid compound and the cholesterol lipid compound ranges from 4:1 to 1:4. In some embodiments, in the liposome for delivery of nucleic acids as described herein, the cationic lipid compounds have an amount from 60wt% to 90wt%, or 70wt% to 90wt%, or 75wt% to 90wt%, or 80wt% to 90wt%, or 85wt% to 90wt%.

The lipid compound as described herein is one or more selected from group consisting of compounds having Formula I, Formula II, Formula III and Formula IV as shown below,

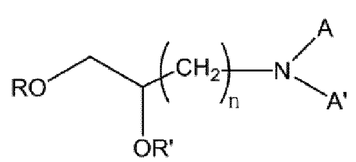

Formula I ;

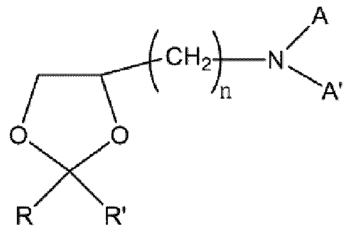

Formula II ;

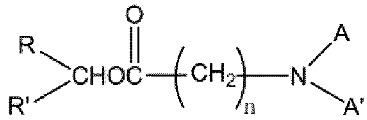

Formula III ;

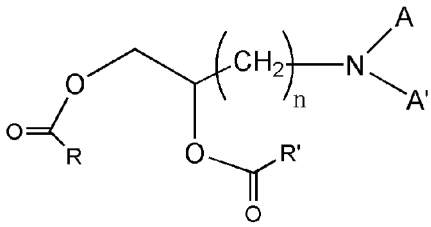

Formula IV ;

wherein, R and R' are independently $C_{14}$-$C_{22}$ saturated or unsaturated fatty acid, A and A' are $C_1$-$C_4$ alkyl, n=1-4.

In the embodiments as described herein, the compounds having Formula I are seleced from group consisting of following compounds:

Compound 1 : R=R'=linoleic acid group, n=1, A=$CH_3$, A'=$CH_3CH_2$ ;
Compound 2 : R=R'=linoleic acid group, n=2, A=$CH_3$, A'=$CH_3CH_2$ ;
Compound 3 : R=R'=linoleic acid group, n=3, A=$CH_3$, A'=$CH_3CH_2$ ;
Compound 4 : R=R'=linoleic acid group, n=4, A=$CH_3$, A'=$CH_3CH_2$ ;
Compound 5 : R=R'=oleic acid group, n=1, A=$CH_3$, A'=$CH_3$ ;
Compound 6 : R=R'=oleic acid group, n=2, A=$CH_3$, A'=$CH_3$ ;
Compound 7 : R=R'=oleic acid group, n=3, A=$CH_3$, A'=$CH_3$ ;
Compound 8 : R=R'=oleic acid group, n=4, A=$CH_3$, A'=$CH_3$ ;
Compound 9 : R=R'=linoleic acid group, n=1, A=$CH_3CH_2$, A'=$CH_3CH_2$ ;
Compound 10 : R=R'=linoleic acid group, n=2, A=$CH_3CH_2$, A'=$CH_3CH_2$ ;
Compound 11 : R=R'=linoleic acid group, n=3, A=$CH_3CH_2$, A'=$CH_3CH_2$ ;
Compound 12 : R=R'=linoleic acid group, n=4, A=$CH_3CH_2$, A'=$CH_3CH_2$ ;
Compound 13 : R=R'=linoleic acid group, n=1, A=$CH_3CH_2$, A'=$CH_3$ ;
Compound 14 : R=R'=linoleic acid group, n=2, A=$CH_3CH_2$, A'=$CH_3$ ;
Compound 15 : R=R'=linoleic acid group, n=3, A=$CH_3CH_2$, A'=$CH_3$ ;
Compound 16 : R=R'=linoleic acid group, n=4, A=$CH_3CH_2$, A'=$CH_3$ ;
Compound 17 : R=R'=oleic acid group, n=1, A=$CH_3CH_2$, A'=$CH_3CH_2$ ;
Compound 18 : R=R'=oleic acid group, n=2, A=$CH_3CH_2$, A'=$CH_3CH_2$ ;
Compound 19 : R=R'=oleic acid group, n=3, A=$CH_3CH_2$, A'=$CH_3CH_2$ ;
Compound 20 : R=R'=oleic acid group, n=4, A=$CH_3CH_2$, A'=$CH_3CH_2$ ;
Compound 21 : R=R'=oleic acid group, n=1, A=$CH_3$, A'=$CH_3CH_2$ ;
Compound 22 : R=R'=oleic acid group, n=2, A=$CH_3$, A'=$CH_3CH_2$ ;

Compound 23 : R=R'=oleic acid group, n=3, A=$CH_3$, A'=$CH_3CH_2$ ;
Compound 24 : R=R'=oleic acid group, n=4, A=$CH_3$, A'=$CH_3CH_2$.

In the embodiments as described herein, the compounds having Formula II are seleced from group consisting of following compounds:

Compound 1' : R=R'=linoleic acid group, n=1, A=$CH_3$, A'=$CH_3CH_2$ ;
Compound 2' : R=R'=linoleic acid group, n=2, A=$CH_3$, A'=$CH_3CH_2$ ;
Compound 3' : R=R'=linoleic acid group, n=3, A=$CH_3$, A'=$CH_3CH_2$ ;
Compound 4' : R=R'=linoleic acid group, n=4, A=$CH_3$, A'=$CH_3CH_2$ ;
Compound 5' : R=R'=oleic acid group, n=1, A=$CH_3$, A'=$CH_3$ ;
Compound 6' : R=R'=oleic acid group, n=2, A=$CH_3$, A'=$CH_3$ ;
Compound 7' : R=R'=oleic acid group, n=3, A=$CH_3$, A'=$CH_3$ ;
Compound 8' : R=R'=oleic acid group, n=4, A=$CH_3$, A'=$CH_3$ ;
Compound 9' : R=R'=linoleic acid group, n=1, A=$CH_3CH_2$, A'=$CH_3CH_2$ ;
Compound 10' : R=R'=linoleic acid group, n=2, A=$CH_3CH_2$, A'=$CH_3CH_2$ ;
Compound 11' : R=R'=linoleic acid group, n=3, A=$CH_3CH_2$, A'=$CH_3CH_2$ ;
Compound 12' : R=R'=linoleic acid group, n=4, A=$CH_3CH_2$, A'=$CH_3CH_2$ ;
Compound 13' : R=R'=linoleic acid group, n=1, A=$CH_3CH_2$, A'=$CH_3$ ;
Compound 14' : R=R'=linoleic acid group, n=2, A=$CH_3CH_2$, A'=$CH_3$ ;
Compound 15' : R=R'=linoleic acid group, n=3, A=$CH_3CH_2$, A'=$CH_3$ ;
Compound 16' : R=R'=linoleic acid group, n=4, A=$CH_3CH_2$, A'=$CH_3$ ;
Compound 13' : R=R'=oleic acid group, n=1, A=$CH_3CH_2$, A'=$CH_3CH_2$ ;
Compound 14' : R=R'=oleic acid group, n=2, A=$CH_3CH_2$, A'=$CH_3CH_2$ ;
Compound 15' : R=R'=oleic acid group, n=3, A=$CH_3CH_2$, A'=$CH_3CH_2$ ;
Compound 16' : R=R'=oleic acid group, n=4, A=$CH_3CH_2$, A'=$CH_3CH_2$ ;
Compound 17' : R=R'=oleic acid group, n=1, A=$CH_3$, A'=$CH_3CH_2$ ;
Compound 18' : R=R'=oleic acid group, n=2, A=$CH_3$, A'=$CH_3CH_2$ ;
Compound 19' : R=R'=oleic acid group, n=3, A=$CH_3$, A'=$CH_3CH_2$ ;
Compound 20' : R=R'=oleic acid group, n=4, A=$CH_3$, A'=$CH_3CH_2$.

In the embodiments as described herein, the compounds having Formula III are seleced from group consisting of following compounds:

Compound 1" : R=R'=linoleic acid group, n=1, A=$CH_3$, A'=$CH_3CH_2$ ;
Compound 2" : R=R'=linoleic acid group, n=2, A=$CH_3$, A'=$CH_3CH_2$ ;
Compound 3" : R=R'=linoleic acid group, n=3, A=$CH_3$, A'=$CH_3CH_2$ ;
Compound 4" : R=R'=linoleic acid group, n=4, A=$CH_3$, A'=$CH_3CH_2$ ;
Compound 5" : R=R'=oleic acid group, n=1, A=$CH_3$, A'=$CH_3$ ;
Compound 6" : R=R'=oleic acid group, n=2, A=$CH_3$, A'=$CH_3$ ;
Compound 7" : R=R'=oleic acid group, n=3, A=$CH_3$, A'=$CH_3$ ;
Compound 8" : R=R'=oleic acid group, n=4, A=$CH_3$, A'=$CH_3$ ;
Compound 9" : R=R'=linoleic acid group, n=1, A=$CH_3CH_2$, A'=$CH_3CH_2$ ;
Compound 10" : R=R'=linoleic acid group, n=2, A=$CH_3CH_2$, A'=$CH_3CH_2$ ;
Compound 11" : R=R'=linoleic acid group, n=3, A=$CH_3CH_2$, A'=$CH_3CH_2$ ;
Compound 12" : R=R'=linoleic acid group, n=4, A=$CH_3CH_2$, A'=$CH_3CH_2$ ;
Compound 13" : R=R'=linoleic acid group, n=1, A=$CH_3CH_2$, A'=$CH_3$ ;
Compound 14" : R=R'=linoleic acid group, n=2, A=$CH_3CH_2$, A'=$CH_3$ ;
Compound 15" : R=R'=linoleic acid group, n=3, A=$CH_3CH_2$, A'=$CH_3$ ;
Compound 16" : R=R'=linoleic acid group, n=4, A=$CH_3CH_2$, A'=$CH_3$ ;
Compound 17" : R=R'=oleic acid group, n=1, A=$CH_3CH_2$, A'=$CH_3CH_2$ ;
Compound 18" : R=R'=oleic acid group, n=2, A=$CH_3CH_2$, A'=$CH_3CH_2$ ;
Compound 19" : R=R'=oleic acid group, n=3, A=$CH_3CH_2$, A'=$CH_3CH_2$ ;
Compound 20" : R=R'=oleic acid group, n=4, A=$CH_3CH_2$, A'=$CH_3CH_2$ ;
Compound 21" : R=R'=oleic acid group, n=1, A=$CH_3$, A'=$CH_3CH_2$ ;
Compound 22" : R=R'=oleic acid group, n=2, A=$CH_3$, A'=$CH_3CH_2$ ;
Compound 23" : R=R'=oleic acid group, n=3, A=$CH_3$, A'=$CH_3CH_2$ ;
Compound 24" : R=R'=oleic acid group, n=4, A=$CH_3$, A'=$CH_3CH_2$.

In the embodiments as described herein, the compounds having Formula IV are seleced from group consisting of following compounds:

Compound 1 ''' : R=R'=linoleic acid group, n=1, A=$CH_3$, A'=$CH_3CH_2$ ;
Compound 2''' : R=R'=linoleic acid group, n=2, A=$CH_3$, A'=$CH_3CH_2$ ;
Compound 3''' : R=R'=linoleic acid group, n=3, A=$CH_3$, A'=$CH_3CH_2$ ;
Compound 4''' : R=R'=linoleic acid group, n=4, A=$CH_3$, A'=$CH_3CH_2$ ;
Compound 5''' : R=R'=oleic acid group, n=1, A=$CH_3$, A'=$CH_3$ ;
Compound 6''' : R=R'=oleic acid group, n=2, A=$CH_3$, A'=$CH_3$ ;
Compound 7''' : R=R'=oleic acid group, n=3, A=$CH_3$, A'=$CH_3$ ;
Compound 8''' : R=R'=oleic acid group, n=4, A=$CH_3$, A'=$CH_3$ ;
Compound 9''' : R=R'=linoleic acid group, n=1, A=$CH_3CH_2$, A'=$CH_3CH_2$ ;
Compound 10''' : R=R'=linoleic acid group, n=2, A=$CH_3CH_2$, A'=$CH_3CH_2$ ;
Compound 11''' : R=R'=linoleic acid group, n=3, A=$CH_3CH_2$, A'=$CH_3CH_2$ ;
Compound 12''' : R=R'=linoleic acid group, n=4, A=$CH_3CH_2$, A'=$CH_3CH_2$ ;
Compound 13''' : R=R'=linoleic acid group, n=1, A=$CH_3CH_2$, A'=$CH_3$ ;
Compound 14''' : R=R'=linoleic acid group, n=2, A=$CH_3CH_2$, A'=$CH_3$ ;
Compound 15''' : R=R'=linoleic acid group, n=3, A=$CH_3CH_2$, A'=$CH_3$ ;
Compound 16''' : R=R'=linoleic acid group, n=4, A=$CH_3CH_2$, A'=$CH_3$ ;
Compound 17''' : R=R'=oleic acid group, n=1, A=$CH_3CH_2$, A'=$CH_3CH_2$ ;
Compound 18''' : R=R'=oleic acid group, n=2, A=$CH_3CH_2$, A'=$CH_3CH_2$ ;
Compound 19''' : R=R'=oleic acid group, n=3, A=$CH_3CH_2$, A'=$CH_3CH_2$ ;
Compound 20''' : R=R'=oleic acid group, n=4, A=$CH_3CH_2$, A'=$CH_3CH_2$ ;
Compound 21''' : R=R'=oleic acid group, n=1, A=$CH_3$, A'=$CH_3CH_2$ ;
Compound 22''' : R=R'=oleic acid group, n=2, A=$CH_3$, A'=$CH_3CH_2$ ;
Compound 23''' : R=R'=oleic acid group, n=3, A=$CH_3$, A'=$CH_3CH_2$ ;
Compound 24''' : R=R'=oleic acid group, n=3, A=$CH_3$, A'=$CH_3CH_2$.

The cholesterol lipid compound as described herein is selected from the group consisting of the compounds having Formula V:

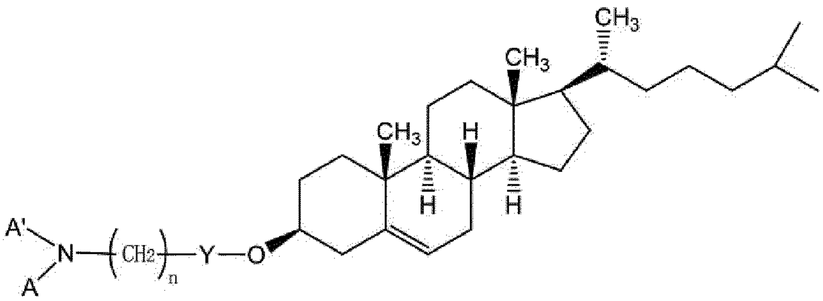

Formula V ;

wherein, Y= (C=O), (C=S), (-HN(O)C-) or bond, A and A' is $C_1$-$C_4$ alkyl, n=1-4.

In the embodiments as described herein, the compounds having Formula V are seleced from group consisting of following compounds:

Compound V1 : Y=(C=O), n=1, A=$CH_3$, A'=$CH_3CH_2$ ;
Compound V2 : Y=(C=O), n=2, A=$CH_3$, A'=$CH_3CH_2$ ;
Compound V3 : Y=(C=O), n=3, A=$CH_3CH_2$, A'=$CH_3CH_2$ ;
Compound V4 : Y=(C=O), n=4, A=$CH_3CH_2$, A'=$CH_3CH_2$ ;
Compound V5 : Y=(C=O), n=1, A=$CH_3CH_2$, A'=$CH_3$ ;
Compound V6 : Y=(C=O), n=2, A=$CH_3$, A'=$CH_3$ ;
Compound V7 : Y=(C=O), n=3, A=$CH_3$, A'=$CH_3$ ;
Compound V8 : Y=(C=O), n=4, A=$CH_3$, A'=$CH_3$ ;
Compound V9 : Y=(C=S), n=1, A=$CH_3CH_2$, A'=$CH_3CH_2$ ;
Compound V10 : Y=(C=S), n=2, A=$CH_3CH_2$, A'=$CH_3CH_2$ ;

Compound V11 : Y=(C=S), n=3, A=$CH_3CH_2$, A'=$CH_3CH_2$ ;
Compound V12 : Y=(C=S), n=4, A=$CH_3CH_2$, A'=$CH_3CH_2$ ;
Compound V13 : Y=(C=S), n=1, A=$CH_3CH_2$, A'=$CH_3$ ;
Compound V14 : Y=(C=S), n=2, A=$CH_3CH_2$, A'=$CH_3$ ;
Compound V15 : Y=(C=S), n=3, A=$CH_3CH_2$, A'=$CH_3$ ;
Compound V16 : Y=(C=S), n=4, A=$CH_3CH_2$, A'=$CH_3$ ;
Compound V17 : Y=(-HNC=O-), n=1, A=$CH_3CH_2$, A'=$CH_3CH_2$ ;
Compound V18 : Y=(-HNC=O-), n=2, A=$CH_3CH_2$, A'=$CH_3CH_2$ ;
Compound V19 : Y=(-HNC=O-), n=3, A=$CH_3CH_2$, A'=$CH_3CH_2$ ;
Compound V20 : Y=(-HNC=O-), n=4, A=$CH_3CH_2$, A'=$CH_3CH_2$ ;
Compound V21 : Y=(-HNC=O-), n=1, A=$CH_3$, A'=$CH_3CH_2$ ;
Compound V22 : Y=(-HNC=O-), n=2, A=$CH_3$, A'=$CH_3CH_2$ ;
Compound V23 : Y=(-HNC=O-), n=3, A=$CH_3$, A'=$CH_3CH_2$ ;
Compound V24 : Y=(-HNC=O-), n=4, A=$CH_3$, A'=$CH_3CH_2$.

In another aspect, a nucleic acid liposome formulation is provided herein, comprising the liposome as described above and a nucleic acid encapsulated within the liposome. The nucleic acid is selected from the group consisting of small interfering nucleic acid, micro nucleic acid, non-coding nucleic acid, antisense nucleic acid, small ligand nucleic acid and small active nucleic acid. The nucleic acid liposome formulation as described herein may further comprise a pharmaceutically acceptable excipient. In one embodiment, the nucleic acid liposome formulation as described herein may be in the form of tablet, or capsule, or lotion, or drop, or power, or solution or aerosol. In another embodiment, the nucleic acid liposome formulation as described herein may be selected from the group consisting of an oral formulation, an intravascular injection formulation, an intramuscular injection formulation, a subcutaneous administration formulation, a parentenral administration formulation, and an intraperitoneal administration formulation.

In yet another aspect, a method for preparing the nucleic acid liposome formulation as mentioned above is provided herein, including:

(1) providing a mixture solution of the cationic lipid compounds, the phospholipid and the PEGylated lipid;
(2) mixing the said mixture solution and the nucleic acid solution to obtain the nucleic acid liposome formulation.

In the mixture solution, the cationic lipid compounds have an amount from 50wt% to 90wt%.

Preferably, after step (1), the mixture solution is subject to extrusion process to obtain empty liposome vesicles. The nucleic acid solution is mixed with the empty liposome vesicles, such that the nucleic acid is loaded into the empty liposome vesicles to form nucleic acid liposome formulation.

In still yet another aspect, a method for treating diseases associated with abnormal expression of genes is provided herein, including administering the nucleic acid liposome formulation as described herein to subjects in need of such treatment. The nucleic acid liposome formulation as described herein is orally, or intravascularly, or intramuscularly, or subcutaneously, or parentemrally or intraperitoneally administered to the patient. In some embodiments as described herein, the nucleic acid encapsulated within the nucleic acid liposome formulation can be delivered into cells, such as cells of target tissues such as liver, inflammation tissues and the like.

The present invention also relates to use of the nucleic acid liposome formulation as described herein in manufacturing a medicament for treating diseases associated with abnormal expression of genes.

The advantages achieved by the present invention comprises:

1) Increased loading capacity. Since the liposome as provided herein comprises the cationic lipid compounds in high amount, the nucleic acid with negative charges can be loaded into the liposome in increased amount, such that the loading capacity for the nucleic acid is greatly increased.
2) Reduced drug toxicity. Since increased nucleic acid drugs can be loaded into the unit lipid vesicles of the liposome formulation, in the case of administration of drug at equivalent dosage, intake of the cationic lipid compounds is relatively decreased due to increased loading capacity, thereby leading to reduced toxicity.

## BRIEF DESCRIPTION OF THE DRAWINGS

Figure 1 is a column diagram of mice body weights before and after administering the liposome formulation with the nucleic acid drug encapsulated therein according to one embodiment as described herein, wherein, the horizontal axis shows respective experiment groups, and the vertical axis shows mice body weights.

Figure 2 is a column diagram of relative mRNA expression level of ApoB gene in mice's liver before and after administering the liposome formulation with the nucleic acid drug encapsulated therein to the mice according to one embodiment as described herein, wherein, the horizontal axis shows respective experiment groups, and the vertical axis shows relative mRNA expression level of ApoB gene.

Figure 3 is a column diagram of total cholesterol level in mice's serum before and after administering the liposome formulation with the nucleic acid drug encapsulated therein to the mice according to one embodiment as described herein, wherein, the horizontal axis shows respective experiment groups, and the vertical axis shows total cholesterol level in mice's serum.

Figure 4 is a column diagram of mice body weights before and after administering the liposome formulation with the nucleic acid drug encapsulated therein to the mice according to another embodiment as described herein, wherein, the horizontal axis shows respective experiment groups, and the vertical axis shows mice body weights.

Figure 5 is a column diagram of relative mRNA expression level of ApoB gene in mice's liver before and after administering the liposome formulation with the nucleic acid drug encapsulated therein to the mice according to another embodiment as described herein, wherein, the horizontal axis shows respective experiment groups, and the vertical axis shows relative mRNA expression level of ApoB gene.

Figure 6 is a column diagram of total cholesterol level in mice's serum before and after administering the liposome formulation with the nucleic acid drug encapsulated therein to the mice according to another embodiment as described herein, wherein, the horizontal axis shows respective experiment groups, and the vertical axis shows total cholesterol level in mice's serum.

Figure 7 is a column diagram of mice body weights before and after administering the liposome formulation with the nucleic acid drug encapsulated therein to the mice according to yet another embodiment as described herein, wherein, the horizontal axis shows respective experiment groups, and the vertical axis shows mice body weights.

Figure 8 is a column diagram of relative mRNA expression level of ApoB gene in mice's liver before and after administering the liposome formulation with the nucleic acid drug encapsulated therein to the mice according to yet another embodiment as described herein, wherein, the horizontal axis shows respective experiment groups, and the vertical axis shows relative mRNA expression level of ApoB gene.

Figure 9 is a column diagram of total cholesterol level in mice's serum before and after administering the liposome formulation with the nucleic acid drug encapsulated therein to the mice according to yet another embodiment as described herein, wherein, the horizontal axis shows respective experiment groups, and the vertical axis shows total cholesterol level in mice's serum.

Figure 10 is a column diagram of mice body weights before and after administering the liposome formulation with the nucleic acid drug encapsulated therein to the mice according to still yet another embodiment as described herein, wherein, the horizontal axis shows respective experiment groups, and the vertical axis shows mice body weights.

Figure 11 is a column diagram of relative mRNA expression level of ApoB gene in mice's liver before and after administering the liposome formulation with the nucleic acid drug encapsulated therein to the mice according to still yet another embodiment as described herein, wherein, the horizontal axis shows respective experiment groups, and the vertical axis shows relative mRNA expression level of ApoB gene.

Figure 12 is a column diagram of total cholesterol level in mice's serum before and after administering the liposome formulation with the nucleic acid drug encapsulated therein to the mice according to still yet another embodiment as described herein, wherein, the horizontal axis shows respective experiment groups, and the vertical axis shows total cholesterol level in mice's serum.

Figure 13 is a column diagram of mice body weights before and after administering the liposome formulation with the nucleic acid drug encapsulated therein to the mice according to still yet another embodiment as described herein, wherein, the horizontal axis shows respective experiment groups, and the vertical axis shows mice body weights.

Figure 14 is a column diagram of relative mRNA expression level of ApoB gene in mice's liver before and after administering the liposome formulation with the nucleic acid drug encapsulated therein to the mice according to still

yet another embodiment as described herein, wherein, the horizontal axis shows respective experiment groups, and the vertical axis shows relative mRNA expression level of ApoB gene.

Figure 15 is a column diagram of total cholesterol level in mice's serum before and after administering the liposome formulation with the nucleic acid drug encapsulated therein to the mice according to still yet another embodiment as described herein, wherein, the horizontal axis shows respective experiment groups, and the vertical axis shows total cholesterol level in mice's serum.

Figure 16 is a column diagram of mice body weights before and after administering the liposome formulation with the nucleic acid drug encapsulated therein to the mice according to still yet another embodiment as described herein, wherein, the horizontal axis shows respective experiment groups, and the vertical axis shows mice body weights.

Figure 17 is a column diagram of relative mRNA expression level of ApoB gene in mice's liver before and after administering the liposome formulation with the nucleic acid drug encapsulated therein to the mice according to still yet another embodiment as described herein, wherein, the horizontal axis shows respective experiment groups, and the vertical axis shows relative mRNA expression level of ApoB gene.

Figure 18 is a column diagram of total cholesterol level in mice's serum before and after administering the liposome formulation with the nucleic acid drug encapsulated therein to the mice according to still yet another embodiment as described herein, wherein, the horizontal axis shows respective experiment groups, and the vertical axis shows total cholesterol level in mice's serum.

## DETAILED DESCRIPTION OF THE INVENTION

Several aspects of the invention are described below in details by reference to appended drawing and specific embodiments. The skilled in the art should understand that the embodiments are set forth to provide an illustration, rather than limit the scope of the present invention. The scope of the present invention is limited by the appended claims.

### Example 1

The lipid compound and the cholesterol lipid compound used in the liposome formulation as described herein were prepared according to the relevant reference documents, patents and patent applications or modified technologies in organic chemistry, synthetic chemistry and biological chemistry fields. For example, the lipid compound (CL52) having Formula I wherein R=R'=linoleic acid group, n=1, A=A'=$CH_3$ was prepared according to the methods that can be found in Heyes J et. al., J Control Release 2005; 107:276-287. The lipid compound (CL53) having Formula I wherein R=R'=oleic acid group, n=1, A=A'=$CH_3$ was prepared according to the methods that can be found in Heyes J et. al., J Control Release 2005; 107:276-287. The lipid compound (CL54) having Formula II wherein R=R'=linoleic acid group, n=2, A=A'=$CH_3$ was prepared according to the methods that can be found in J Chen et al., PCT applications WO/2009/086558 and WO/2010/042877 or Semple SC et al., Nature Biotech, 2010; 28:172-176. The lipid compound (CL51) having Formula III wherein R=R'=linoleic acid group, n=3, A=A'=$CH_3$ was prepared according to the methods that can be found in Jayaraman M et al., Angew Chem Int Ed Engl. 2012;51:8529-33. The lipid compound (CL55) having Formula IV wherein R=R'=linoleic acid group, n=1, A=A'=$CH_3$ was prepared according to the methods that can be found in Leventis R et al., Biochim Biophys Acta 1990; 1023:124-132. The lipid compound (CL56) having Formula IV wherein R=R'=oleic acid group, n=1, A=A'=$CH_3$ was prepared according to the methods that can be found in Leventis R et al., Biochim Biophys Acta 1990; 1023:124-132. PEG-c-DMA was prepared according to the methods that can be found in J Chen et al., PCT applications WO/2009/086558 and WO/2010/042877 or Semple SC et al., Nature Biotech, 2010; 28:172-176.

The cholesterol lipid compound (CL66) having Formula V wherein Y=(C=O), A=A'=$CH_3$, n=2 was prepared as follows.

Cholesterol (1.1g, 2.84 mmol) dissolved in DCM (20mL) and material 11 (4-(dimethylamino)propionic acid hydrochloride, 0.7g, 4.55mmol) were added into 100mL flask and then di-isopropyl ethylamine (1mL) and DMAP (0.056g, 0.56mmol) were added thereto. The mixture in the flask was stirred for 5 min at room temperature and EDC·HCl (1.1g, 5.6mmol) was added and stirred overnight at room temperature. Progresses of reaction was detected by TLC (5% MeOH/DCM). After completing the reaction, 20 mL DCM was added to dilute the solution. 16 mL saturated $NaHCO_3$, 16mL water and 16 mL saturated saline were used to wash the solution. The organic phase was dried over anhydrous $Na_2SO_4$, filtered and concentrated, to yield 1.1 g crude product. The crude product was purified by using Flash column (90g silica gel, loading by 210 mL DCM containing 0.1% TEA; the moving phases were respectively: 140 mL DCM containing 0.1% TEA, 560 ml 2% methanol+98% DCM containing 0.1% TEA; 140 mL 2.5% methanol + 97.5% DCM containing 0.1% TEA, 420 mL 3% methanol +97% DCM containing 0.1 TEA) to yield purified product CL66.

The cholesterol lipid compound (CL68) having Formula V wherein Y=(C=O), A=A'=$CH_3$, n=1 was prepared as follows.

Cholesterol (1.1g, 2.84 mmol) dissolved in DCM (20mL) and material 13 (4-(dimethylamino)propionic acid hydrochloride, 0.7 g, 5.0 mmol) were added into 100mL flask and then di-isopropyl ethylamine (1mL) and DMAP (0.056g, 0.56mmol) were added thereto. The mixture in the flask was stirred for 5 min at room temperature and EDC HCl (1.1g, 5.6mmol) was added and stirred overnight at room temperature. Progresses of reaction were detected by TLC (5% MeOH/DCM). After completing the reaction, 20 mL DCM was added to dilute the solution. 16 mL saturated $NaHCO_3$, 16mL water and 16 mL saturated saline were used to wash the solution. The organic phase was dried over anhydrous $Na_2SO_4$, filtered and concentrated, to yield 1.1 g crude product. The crude product was purified by using Flash column (90g silica gel, loading by 210 mL DCM containing 0.1% TEA; the moving phases were repectively: 140 mL DCM containing 0.1% TEA, 560 ml 2% methanol +98% DCM with containing 0.1% TEA; 140 mL 2.5% methanol+97.5% DCM containing 0.1% TEA, 420 mL 3% methanol + 97% DCM containing 0.1% TEA) to yield purified product CL68.

**Example 2**

Preparation of Liposome Formulation

1. Reagents, Materials and Instruments

1.1 Reagents:

The lipid compound was compound CL51 having Formula III wherein R=R'=linoleic acid group and n=3.

The cholesterol lipid compounds were compounds CL66 and CL68 prepared according to Example 1.

The phospholipid was distearoyl phosphatidylcholine (DSPC) purchased from Shanghai Advanced Vehicle Technology L.T.D. Co.

The PEGylated lipid was PEG-c-DMA prepared according to Example 1.

The fluorescent dye, potassium 6-(p-toluidino)-2-naphthalenesulphonate (TNS), was purchased from Sigma-Aldrich Co.

Other reagents including absolute ethanol, methanol, chloroform, citric acid, sodium citrate, NaCl and so on were imported or domestic analytical grade reagents.

The exemplary nucleic acid medicament used in the present example was ApoB-siRNA synthesized by BIOMICS BIOTECHNOLOGIES CO., LTD. in the form of freeze-dried powder, with following sequence:

ApB-S : 5'-GUCAUCACACUGAAUACCAAU-3' ;
ApB-AS : 5'-AUUGGUAUUCAGUGUGAUGACAC-3'.

1.2 Materials: ion exchanging column: Vivapure D Mini H (Sartorius stedim Co., USA), Ultracel-100 centrifugal ultrafiltration tube (Millipore Co., USA), dialysis membrane: Nuclepore Membrane Circles (80nm) (Whatman Co., USA), dialysis bag and needle filter purchased from Sangon Biotech Co. Ltd., dialysis bag MD24 (MW: 20000, Shanghai Baoman Biotechnology Co. Ltd.), needle filter (Sangon Biotech Co. Ltd.), 50mL Falcon tube (Corning Co., USA).

1.3 Instruments: Vortex Mixer (VWR Co., USA), constant temperature water bath (Jiangsu Tianhong Instrument Co.), Centrifuge (Eppendorf Co., USA), UV-Vis Spectrophotometer (Shanghai Jingke Co.), Magnetic Stirrer (Shanghai Meiyingpu Instrument Co.), NLI Liposome Extruder (ATS Co., CA), Clean Bench (Suzhou Jinghua), High Speed Refrigerated Centrifuge (Saite Xiangyi), Fluorospectro Photometer (RF-5301PC, Shimadzu Co., JP) and so on.

2. Preparation of liposome formulation

2.1 Preparation of Reagents:

2.1.1 Preparation of lipid stock solution

The lipid compound CL51, the cholesterol lipid compounds CL66 and CL68, the phospholipid DSPC, and the PEGylated lipid PEG-c-DMA were balanced at room temperature for about 30 min before weighting.

The lipid compound CL51 was formulated to 40mg/mL stock solution by using 100% ethanol. The cholesterol lipid compounds CL66 and CL68 were formulated to 20mg/mL stock solution by using 100% ethanol, respectively. DSPC was formulated to 20mg/mL stock solution by using 100% ethanol. The PEGylated lipid PEG-c-DMA was formulated to 50mg/mL stock solution by using 100% ethanol.

2.2 Preparation of liposome formulations

The liposome formulations F61-F520 were prepared at the ratio between the lipid compound CL51 and the cholesterol lipid compound CL68 as listed in Table 1 (wherein, the PEGylated lipid was PEG-c-DMA at amount of 10% and the remainder was DSPC to arrive at 100%, and the amounts of the PEGylated lipid and the phospholipid as mentioned above were used in following examples). In particular, the lipid compound CL51, the cholesterol lipid compound CL68, the PEGylated lipid PEG-c-DMA and phospholipid DSPC at the ratios as listed in table 1 were formulated into ethanol solution by using absolute ethanol as solvent. After all of the lipids were dissolved, the ethanol solution of the lipids as mentioned above was added into buffer solution (50mM ctric acid, pH 4.0) with stirring, to obtain 30% (v/v) of final ethanol concentration. At room temperature, under pressure of 200 psi, extrusion was performed by using NLI liposome extruder equipped with two layers of Nuclepore Membrane Circles. Empty liposome vesicles with uniform diameter were obtained. The diameter of the empty liposome vesicles obtained through extruder was measured by particle size analyzer.

Table 1.

| Formulation | Amount of cationic lipid compounds (%) | Lipid compound: Cholesterol lipid compound | Lipid compound | Cholesterol lipid compound |
|---|---|---|---|---|
| F601 | 60 | 4:1 | CL51 | CL68 |
| F602 | 60 | 2:2 | CL51 | CL68 |
| F603 | 60 | 1:4 | CL51 | CL68 |
| F608 | 60 | 2:1 | CL51 | CL68 |
| F609 | 60 | 2:1 | CL51 | CL66 |
| F610 | 60 | 1:1 | CL51 | CL66 |
| F504 | 70 | 4:1 | CL51 | CL68 |
| F505 | 75 | 4:1 | CL51 | CL68 |
| F506 | 80 | 4:1 | CL51 | CL66 |
| F507 | 80 | 2:2 | CL51 | CL68 |
| F510 | 80 | 1:4 | CL51 | CL68 |
| F511 | 83 | 4:1 | CL51 | CL68 |
| F512 | 85 | 4:1 | CL51 | CL68 |
| F513 | 88 | 4:1 | CL51 | CL68 |
| F518 | 90 | 4:1 | CL51 | CL68 |
| F519 | 90 | 2:2 | CL51 | CL66 |
| F520 | 90 | 1:4 | CL51 | CL66 |

2.3 Preparation of liposome formulation with siRNA encapsulated therein

2.3.1 Preparation of siRNA stock solution:

Dissolution of siRNA: ApoB-siRNA stock solution with 50mg/mL of theorical final concentration was formulated by using solution of 10 mM citric acid / 30mM NaCl at pH of 6.0. ApoB-siRNA stock solution in a suitable amount was diluted 2000 times by PBS for detection of concentration of siRNA. Each sample was repeated in triplicate. $A_{260}$ value

was detected by UV-Vis Spectrophotometer to calculate actual concentration of ApoB-siRNA stock solution.

The empty liposome vesicles at desired volume was added into 50 mL Falcon tube to prebalance in water bath at 35°C for 4~5min. The ApoB-siRNA stock solution was formulated to siRNA/formulation buffer/ethanol solution to make sure that ethanol was comprised of 30% relative to total volume. The siRNA/formulation buffer/ethanol solution was slowly added to the empty liposome vesicles and incubated at 35°C for 30min. After incubation, 600 μL incubated product was used for $A_{260}$ analysis. The siRNA liposome formulation was subject to dialysis overnight by using dialysis bag MD24 in 1 x PBS. The dialyzed product was recovered and filtered by using a 0.2 μm needle filter to remove bacteria. After removing bacteria, 600 μL product was used for detection of encapsulation efficiency.

2.4 Detection of encapsulation efficiency

300 μL product from which bacteria was removed passes through Vivapure D Mini H (ion exchange column), to obtain post-chromatography sample. The remainder 300 μL product from which bacteria was removed was used as the sample without chromatography. The actual concentration coefficient (mg/mL) was calculated according to the actual $A_{260}$ value, i.e., the actual concentration coefficient (mg/mL) = volume of the added ApoB-siRNA stock solution (mL) x actual concentration of ApoB-siRNA stock solution (mg/mL) x 0.03 (mL) x 1000 / (total volume of siRNA liposome formulation (mL) x $A_{260}$ value of incubated product x 1.1 (mL)). The encapsulation efficiency was calculated according to siRNA concentration. The encapsulation efficiency can be defined as encapsulation efficiency = (the medicaments encapsulated in the liposome/total amount of the medicaments in the liposome formulation) x 100%. The encapsulation efficiency can be calculated according to the equation: encapsulation efficiency = siRNA concentration of the post-chromatography sample / siRNA concentration of the sample without chromatography.

2.5 Loading efficiency = (the amount of the medicaments encapsulated in the liposome/liposome + total amount of the medicaments in the liposome formulation) x 100%.

2.6 Detection of pKa of the empty liposome vesicles

The fluorescent dye TNS radiated no luminescence or weak luminescence in aqueous solution, while it radiated strong luminescence in organic environment. pKa value of the empty liposome vesicles can be detected by taking advantage of fluorescent characteristics of TNS. When TNS with negative charges was added into the solution of cationic liposomes, it aggregated on the surfaces of the liposomes to radiate fluorescence; while free TNS in the aqueous solution radiated no luminescence. Therefore, the fluorescent density of the solution (within a certain concentration range) exhibited positive proportion to the amount of the positive charges on the surfaces of the liposomes. As such, ionization extent of amino in the cation liposomes can be obtained by detecting the fluorescent density. According to the definition of the dissociation constant, when the ionization extent of the weak acid in the solution arrived at 50%, the pH value of the solution was the pKa value of the weak acid. Therefore, according to the diagram of the fluorescent density relative to the pH value, pH value corresponding to the middle point of the fluorescent density was the apparent pKa value of the cation solution.

The particular detection method included:

1) adding 2 mL PBS solution to each sample flask and adjusting pH values of PBS solution in each sample flask to various pH values (2.5, 3.0, 3.5, 4.0, 4.5, 5.0, 5.5, 6.0, 6.5, 7.0, 7.5, 8.0, 8.5, 9.0, 9.5, 10.0, 10.5, 11.0 respectively) by HCl and NaOH;
2) adding to each sample flask 80 μL 250mM 4-morpholine ethanesulfonic acid solution, 80μL 250mM HEPES solution, 80 μL 250mM ammonium acetate solution, 80 μL 3.85 M sodium chloride solution and 60 μL PFV in turn;
3) adding 40mL 83.5μM TNS solution to the sample flask containing PBS solution at pH value of 2.5 and stirring for 30s, then removing the solution and measuring the fluorescent density thereof by using fluorospectro photometer (excitation wavelength is 321nm, emission wavelength is 445nm);
4) according to the steps as mentioned above, measuring fluorescent density of TNS under various pH values, plotting a diagram with horizontal axis of pH value and vertical axis of fluorescent density, wherein pH value corresponding to the middle point of the fluorescent density is apparent pKa value of PFV, thereby obtaining pKa value.

2.7 Analysis of particle size

Particle sizes of the empty liposome vesicles and siRNA liposome formulations were measured by Nicomp 370 particle size analyser.

Table 2 listed the particle sizes, pKa values of the empty liposome vesicles prepared as mentioned above and encapsulation efficiencies and loading efficiencies of the empty liposome vesicles for siRNA.

Table 2.

| Formulation | Particle Size (nm) | pKa Value | Encapsulation Efficiency for siRNA | Loading Efficiencies for siRNA |
|---|---|---|---|---|
| F601 | 63.35 | 5.77 | 65.9 | 68.9 |
| F602 | 62.01 | 6.01 | 66.1 | 67.3 |
| F603 | 65.09 | 5.93 | 63.4 | 69.4 |
| F608 | 63.15 | 6.55 | 67.9 | 65.2 |
| F609 | 69.31 | 7.32 | 55.4 | 68.3 |
| F610 | 68.13 | 7.45 | 36.6 | 69.0 |
| F504 | 69.35 | 6.31 | 71.5 | 70.3 |
| F505 | 66.29 | 7.32 | 69.3 | 78.7 |
| F506 | 65.49 | 7.51 | 65.5 | 76.3 |
| F507 | 68.75 | 6.55 | 69.7 | 77.9 |
| F510 | 69.33 | 6.54 | 70.9 | 78.6 |
| F511 | 65.29 | 5.98 | 65.3 | 79.8 |
| F512 | 69.54 | 6.11 | 68.4 | 80.8 |
| F513 | 78.11 | 6.87 | 67.9 | 81.3 |
| F518 | 79.21 | 6.73 | 70.2 | 87.5 |
| F519 | 81.32 | 5.99 | 69.5 | 85.1 |
| F520 | 80.10 | 6.31 | 71.3 | 88.0 |

From the measurements of encapsulation efficiencies and loading efficiencies for siRNA as listed in Table 2, it can be seen that the encapsulation efficiencies and loading efficiencies for siRNA were increased as increase of the content of cationic lipid compounds in the liposome formulation. The nucleic acid with negative charges loaded into the liposome formulation was increased due to increase of the content of the cationic lipid compounds. Therefore, loading amount for the nucleic acid medicaments can be greatly increased by using the liposome formulation as described herein. As a result, increased amount of nucleic acid medicaments can be loaded into the unit lipid vesicles of the liposome formulation as described herein. In the case of administration of medicaments at the same dosage, intake of the cationic lipid compounds was decreased due to increased loading amount for the nucleic acid medicaments, so as to decrease use of lipid and toxicity.

**Example 3**

Efficiency of the liposome formulation

1. Reagents, Materials and Instruments

1.1 Reagents and Materials: liposome formulation (as prepared according to Example 2), sodium chloride injection solution (Shandong Kangning Pharmaceutical Co. Ltd.), RISO™ RNA extraction reagent (BIOMICS BIOTECHNOLOGIES CO., LTD), 1mL sterilized syringe (Henan Shuguangjianshi Medical Device Co. Ltd.), total cholesterol (CHO enzyme test) detection kit (Nanjing Jiancheng Biology Co.).

1.2 Experiment Animals: 4-6 weeks female ICR mice, 18-22g, purchased from Comparison Medical Center of Nantong University.

1.3 Instruments: LightCycler 480 Real-time fluorescence quantification PCR analyser (Roche Co., USA), UV-Vis Spectrophotometer UV759 (Shanghai Jingke Co.).

2. Experimental Method

2.1 Experimental Grouping: weighting mice body weight before administration and grouping the mice according to the body weight into 3 groups: siRNA liposome formulation treated gourp (Table 1), saline treated group.
siRNA liposome formulation treated gourp: the injection amount was calculated according to 3mg/kg of siRNA in the liposome;
saline treated group: 300 μL sodium chloride injection solution.

2.2 Administration: fixing mice by fixer and injecting liposome formulation in an amount of 3mg/kg siRNA in the liposome formulation via tail vein of mice using 1mL sterilized syringe; injecting 300μL sodium chloride injection solution for control group.

2.3 Testing of total cholesterol content in serum of mice
Mice body weight was weighted at 48h upon administration. About 800 μL of blood of mice was collected by removing eyes and placed at 4°C for 1h. Serum was separated by centrifugation at 3000rpm for 10min for test. Total cholesterol in 10μL separated mice serum was detected according to the instruction of the total cholesterol (CHO enzyme test) detection kit and absorbancy at 500nm was detected.
Cholesterol content was calculated according to the equation that cholesterol content = ($A_{500}$ value of the testing sample/$A_{500}$ value of the standard sample) x concentration of standard solution (200mg/dL (5.17mM)).

2.4 Real-time quantification PCR (RT-qPCR) was used to detect mRNA expression level of ApoB gene in mice liver.

At 48h upon injection of liposome formulation, mice were sacrificed and three blocks of tissues from various positions in liver were removed from mice. Total RNA of mice liver was extracted by using RISO™ RNA extraction reagent according to the manufacture's instruction. mRNA expression level of ApoB gene in mice liver was detected by RT-qPCR.

mRNA expression level of ApoB gene in the sample was detected by using primer with gene specificity and housekeeping gene GAPDH was amplified for internal reference. ApoB gene and internal reference gene GAPDH for each sample were amplified at the same time in triplicate. OneStep qPCR kit was used for quantification of reaction. The following reaction system was established: 2μL RNA template, 12.5μL 2 x Master Mix, 0.5μL for each 5'-end primer (10μM) and 3'-end primer (10μM), 0.5μL 50 x SYBR Green I Solution. The volume of the system was supplemented to 25 μL by using water without RNase. After mixing, the system was placed on the quantification PCR analyser for reaction.

The sequence of 5'-end primer for detection of mRNA of ApoB gene was AAGCACCTCCGAAAGTACGTG, and the sequence of 3'-end primer for detection of mRNA of ApoB gene is CTCCAGCTCTACCTTACAGTTGA. The sequence of 5'-end primer for detection of the housekeeping gene GAPDH was GTATGACTCCACTCACGGCAAA and the sequence of 3'-end primer for detection of the housekeeping gene GAPDH was GGTCTCGCTCCTGGAAGATG. All of the primers were synthesized by BIOMICS BIOTECHNOLOGIES CO., LTD.

Reaction condition comprises: reverse transcription for 30 min at 42°C, initial denaturation for 5 min at 95°C, denaturation for 20sec at 95°C, anneal for 30sec at 58°C, extension for 30sec at 72°C, circulation for 45 times. Dissolution curve reaction is performed under95°C/5min, 58°C/5min and the temperature is increased to 95°C at 0.5°C/5sec.

2.5 Statistical Analysis

SPSS 14.0 statistical software was used and the data was shown as $\bar{x}\pm s$. Significant difference among groups was detected by single factor variance analysis. Comparison between two groups was performed by t test. $P$ value < 0.05 was considered to be statistically significant.

3. Experimental Results

3.1 Variation of mice body weight. Variation of mice body weight before and after injection of liposome formulation was detected to indirectly reflect toxicity. As shown in Figure 1, no significant variation of mice body weight was shown in each group before and after administration, indicating that the liposome formulation as described herein had no significant toxicity.

3.2 Variation of mRNA level of ApoB gene. As shown in Figure 2, the liposome forumation as described herein effectively inhibited expression of ApoB mRNA in liver.

3.3 Variation of total cholesterol content. As shown in Figure 3, in comparision with the control group, the total

cholesterol level in serum of mice was decreased by using the liposome formulation as described herein.

In sum, it can be seen that the liposome formulation of the present invention was able to deliver nucleic acid medicaments such as siRNA to target tissues or organs so as to facilitate the nucleic acid medicaments to play their role.

**Example 4**

1. Preparation of liposome formulation with siRNAs encapsulated therein

1.1 The cationic lipid compounds consisted of lipid compound CL52 and cholesterol lipid compound CL68. Phospholipid was DSPC purchased from Shanghai Shanghai Advanced Vehicle Technology L.T.D. Co. The PEGylated lipid was PEG-c-DMA.

1.2 The liposome formulation with siRNAs encapsulated therein was prepared according to the steps as described in Example 2 and parameters were detected. The ratios between lipid compounds were listed in Table 3. Table 4 listed particl sizes and pKa values of the liposome formulations and the encapsulation efficiencies and loading efficiencies of the liposome formulations with siRNA encapsulated therein.

Table 3.

| Formulations | Content of cationic lipid compounds (%) | Lipid lipid compound: Cholesterol lipid compound | Lipid compound | Cholesterol lipid compound |
|---|---|---|---|---|
| F611 | 40 | 2:1 | CL52 | CL68 |
| F612 | 60 | 2:1 | CL52 | CL68 |
| F613 | 80 | 2:1 | CL52 | CL68 |
| F614 | 90 | 2:1 | CL52 | CL68 |

Table 4.

| Formulations | Particle Size (nm) | pKa value | siRNA Encapsulation Efficiency | siRNA Loading Efficiency |
|---|---|---|---|---|
| F611 | 88.30 | 6.52 | 66.3 | 48.1 |
| F612 | 85.49 | 6.87 | 60.8 | 67.3 |
| F613 | 79.63 | 6.51 | 79.3 | 79.5 |
| F614 | 69.97 | 6.79 | 75.6 | 87.9 |

From the above table 4, it can be seen that siRNA encapsulation efficiency and loading efficiency were increased with increase of the content of cationic lipid compounds in the liposome formulation. This indicated that the nucleic acid with negative charges loaded into the liposome was increased due to the increase of the the content of cationic lipid compounds. Therefore, the loading efficiency for nucleic acid medicaments was greatly increased by using the liposome formulation as described herein. Therefore, increased nucleic acid medicament was loaded into the unit lipid vesicles of the liposome formulation as described herein. At the same dosage, intake of cationic lipid compounds was descreased due to increased loading amount, thereby reducing use of the lipid and toxicity.

2. Efficiency of the liposome formulation with siRNAs encapsulated therein

The efficiency of the liposome formulation with siRNAs encapsulated therein as prepared in this example was detected according to the method as described in Example 3.

3. Experimental Results

3.1 Variation of mice body weight. As shown in Figure 4, no significant variation of mice body weight was shown in each group before and after administration, indicating that the liposome formulation as described herein had no significant toxicity.

3.2 Variation of mRNA level of ApoB. As shown in Figure 5, the liposome forumation as described herein effectively inhibited expression of ApoB mRNA in liver.

3.3 Variation of total cholesterol content. As shown in Figure 6, in comparision with control group, the total cholesterol level in serum of mice was decreased by using the liposome formulation as described herein.

In sum, it can be seen that the liposome formulation of the present invention was able to deliver nucleic acid medicaments such as siRNA to target tissues or organs so as to facilitate the nucleic acid medicaments to play their role.

**Example 5**

1. Preparation of liposome formulation with siRNAs encapsulated therein

1.1 The cationic lipid compounds consisted of lipid compound CL53 and cholesterol lipid compound CL68. Phospholipid was DSPC purchased from Shanghai Advanced Vehicle Technology L.T.D. Co. The PEGylated lipid was PEG-c-DMA.

1.2 The liposome formulation with siRNAs encapsulated therein was prepared according to the steps as described in Example 2. The lipid compounds were listed in Table 5. Table 6 listed particle sizes and pKa values of the liposome formulations and the encapsulation efficiencies and loading efficiencies of the liposome formulations with siRNA encapsulated therein.

Table 5.

| Formulation | Content of cationic lipid compounds (%) | Lipid compound: Cholesterol lipid compound | Lipid compound | Cholesterol lipid compound |
|---|---|---|---|---|
| F615 | 40 | 2:1 | CL53 | CL68 |
| F616 | 60 | 2:1 | CL53 | CL68 |
| F617 | 80 | 2:1 | CL53 | CL68 |
| F618 | 90 | 2:1 | CL53 | CL68 |

Table 6.

| Formulation | Particle Size (nm) | pKa value | siRNA Encapsulation Efficiencies | siRNA Loading Efficiencies |
|---|---|---|---|---|
| F615 | 68.12 | 6.55 | 56.5 | 51.3 |
| F616 | 65.56 | 6.67 | 60.8 | 65.3 |
| F617 | 69.34 | 6.59 | 69.5 | 78.5 |
| F618 | 67.37 | 6.63 | 67.7 | 89.3 |

From the above table 6, it can be seen that siRNA encapsulation efficiency and loading efficiency were increased with increase of the content of cationic lipid compounds in the liposome formulation. This indicated that the nucleic acid with negative charges loaded into the liposome was increased due to increase of the content of the cationic lipid compounds. Therefore, the loading efficiency for nucleic acid medicaments was greatly increased by using the liposome formulation as described herein. As such, increased nucleic acid medicament was loaded into the unit lipid vesicle of the liposome formulation as described herein. At the same dosage, intake of cationic lipid compounds was descreased due to increased loading amount, thereby reducing use of the lipid and toxicity.

2. Efficiency of the liposome formulation with siRNAs encapsulated therein

The efficiency of the liposome formulation with siRNAs encapsulated therein as prepared in this example was

detected according to the method as described in Example 3.

3. Experimental Results

3.1 Variation of mice body weight. As shown in Figure 7, no significant variation of mice body weight was shown in each group before and after administration, indicating that the liposome formulation as described herein had no significant toxicity.

3.2 Variation of mRNA level of ApoB. As shown in Figure 8, the liposome forumation as described herein effectively inhibited expression of ApoB mRNA in liver.

3.3 Variation of total cholesterol content. As shown in Figure 9, in comparision with control group, the total cholesterol level in serum of mice was decreased by using the liposome formulation as described herein.

In sum, it can be seen that the liposome formulation of the present invention was able to deliver nucleic acid medicaments such as siRNA to target tissues or organs so as to facilitate the nucleic acid medicaments to play their role.

**Example 6**

1. Preparation of liposome formulation with siRNAs encapsulated therein

1.1 The cationic lipid compounds consisted of lipid compound CL54 and cholesterol lipid compound CL68. Phospholipid was DSPC purchased from Shanghai Advanced Vehicle Technology L.T.D. Co. The PEGylated lipid was PEG-c-DMA.

1.2 The liposome formulation with siRNAs encapsulated therein was prepared according to the steps as described in Example 2. The ratios between two lipid compounds were listed in Table 7. Table 8 listed particle sizes and pKa values of the liposome formulations and the encapsulation efficiencies and loading efficiencies of the liposome formulations with siRNAs encapsulated therein.

Table 7.

| Formulation | Content of cationic lipid compounds (%) | Lipid lipid compound : Cholesterol lipid compound | Lipid compound | Cholesterol lipid compound |
|---|---|---|---|---|
| F619 | 40 | 2:1 | CL54 | CL68 |
| F620 | 60 | 2:1 | CL54 | CL68 |
| F621 | 80 | 2:1 | CL54 | CL68 |
| F622 | 90 | 2:1 | CL54 | CL68 |

Table 8.

| Formulation | Particle Size (nm) | pKa Value | siRNA Encapsulation Efficiency | siRNA Loading Efficiency |
|---|---|---|---|---|
| F619 | 78.29 | 7.51 | 66.3 | 49.5 |
| F620 | 83.33 | 6.97 | 50.2 | 55.3 |
| F621 | 79.49 | 6.83 | 59.5 | 68.5 |
| F622 | 77.73 | 6.68 | 61.9 | 79.8 |

From the above table 8, it can be seen that siRNA encapsulation efficiency and loading efficiency were increased with increase of the content of cationic lipid compounds in the liposome formulation. This indicated that the nucleic acid with negative charges loaded into the liposome was increased due to increase of the content of the cationic lipid compounds. Therefore, the loading efficiency for nucleic acid medicaments was greatly increased by using the liposome

formulation as described herein. As such, increased nucleic acid medicament was loaded into the unit lipid vesicles of the liposome formulation as described herein. At the same dosage, intake of cationic lipid compounds was descreased due to increased loading amount, thereby reducing use of the lipid and toxicity.

2. Efficiency of the liposome formulation with siRNAs encapsulated therein

The efficiency of the liposome formulation with siRNAs encapsulated therein as prepared in this example was detected according to the method as described in Example 3.

3. Experimental Results

3.1 Variation of mice body weight. As shown in Figure 10, no significant variation of mice body weight was shown in each group before and after administration, indicating that the liposome formulation as described herein had no significant toxicity.

3.2 Variation of mRNA level of ApoB. As shown in Figure 11, the liposome forumation as described herein effectively inhibited expression of ApoB mRNA in liver.

3.3 Variation of total cholesterol content. As shown in Figure 12, in comparision with control group, the total cholesterol level in serum of mice was decreased by using the liposome formulation as described herein.

In sum, it can be seen that the liposome formulation of the present invention was able to deliver nucleic acid medicaments such as siRNA to target tissues or organs so as to facilitate the nucleic acid medicaments to play their role.

**Example 7**

1. Preparation of liposome formulation with siRNAs encapsulated therein

1.1 The cationic lipid compounds consisted of lipid compound CL55 and cholesterol lipid compound CL68. Phospholipid was DSPC purchased from Shanghai Advanced Vehicle Technology L.T.D. Co.. The PEGylated lipid was PEG-c-DMA.

1.2 The liposome formulation with siRNAs encapsulated therein was prepared according to the steps as described in Example 2. The ratios between two lipid compounds were listed in Table 9. Table 10 listed particle sizes and pKa values of the liposome formulations and the encapsulation efficiencies and loading efficiencies of the liposome formulations with siRNAs encapsulated therein.

Table 9.

| Formulation | Content of cationic lipid compounds (%) | Lipid compound: cholesterol lipid compound | Lipid compound | cholesterol lipid compound |
|---|---|---|---|---|
| F623 | 40 | 2:1 | CL55 | CL68 |
| F624 | 60 | 2:1 | CL55 | CL68 |
| F625 | 80 | 2:1 | CL55 | CL68 |
| F626 | 90 | 2:1 | CL55 | CL68 |

Table 10.

| Formulation | Particle Size (nm) | pKa Value | siRNA Encapsulation Efficiency | siRNA Loading Efficiency |
|---|---|---|---|---|
| F623 | 68.93 | 6.95 | 58.3 | 46.3 |
| F624 | 73.13 | 6.93 | 65.2 | 58.3 |

(continued)

| Formulation | Particle Size (nm) | pKa Value | siRNA Encapsulation Efficiency | siRNA Loading Efficiency |
|---|---|---|---|---|
| F625 | 76.64 | 6.53 | 69.5 | 63.2 |
| F626 | 79.31 | 6.48 | 71.9 | 89.8 |

From the above table 10, it can be seen that siRNA encapsulation efficiency and loading efficiency were increased with increase of the content of cationic lipid compounds in the liposome formulation. This indicated that the nucleic acid with negative charges loaded into the liposome was increased due to increase of the content of the cationic lipid compounds. Therefore, the loading efficiency for nucleic acid medicaments was greatly increased by using the liposome formulation as described herein. As such, increased nucleic acid medicament was loaded into the unit lipid vesicle of the liposome formulation. At the same dosage, intake of cationic lipid compounds was descreased due to increased loading amount, thereby reducing use of the lipid and toxicity.

2. Efficiency of the liposome formulation with siRNA encapsulated therein

The efficiency of the liposome formulation with siRNA encapsulated therein as prepared in this example was detected according to the method as described in Example 3.

3. Experimental Results

3.1 Variation of mice body weight. As shown in Figure 13, no significant variation of mice body weight was shown in each group before and after administration, indicating that the liposome formulation as described herein had no significant toxicity.

3.2 Variation of mRNA level of ApoB. As shown in Figure 14, the liposome forumation as described herein effectively inhibited expression of ApoB mRNA in liver.

3.3 Variation of total cholesterol content. As shown in Figure 15, in comparision with control group, the total cholesterol level in serum of mice was decreased by using the liposome formulation as described herein.

In sum, it can be seen that the liposome formulation of the present invention was able to deliver nucleic acid medicaments such as siRNA to target tissues or organs so as to facilitate the nucleic acid medicaments to play their role.

**Example 8**

1. Preparation of liposome formulation with siRNAs encapsulated therein

1.1 The cationic lipid compounds consisted of lipid compound CL56 and cholesterol lipid compound CL68. Phospholipid was DSPC purchased from Shanghai Advanced Vehicle Technology L.T.D. Co.. The PEGylated lipid was PEG-c-DMA.

1.2 The liposome formulation with siRNAs encapsulated therein was prepared according to the steps as described in Example 2. The ratios between two lipid compounds were listed in Table 11. Table 12 listed particle sizes and pKa values of the liposome formulations and the encapsulation efficiencies and loading efficiencies of the liposome formulations with siRNAs encapsulated therein.

Table 11.

| Formulation | Content of cationic lipid compounds (%) | Lipid compound: Cholesterol lipid compound | Lipid compound | Cholesterol lipid compound |
|---|---|---|---|---|
| F627 | 40 | 2:1 | CL56 | CL68 |

(continued)

| Formulation | Content of cationic lipid compounds (%) | Lipid compound: Cholesterol lipid compound | Lipid compound | Cholesterol lipid compound |
|---|---|---|---|---|
| F628 | 60 | 2:1 | CL56 | CL68 |
| F629 | 80 | 2:1 | CL56 | CL68 |
| F630 | 90 | 2:1 | CL56 | CL68 |

Table 12.

| Formulation | Particle Size (nm) | pKa Value | siRNA Encapsulation Efficiency | siRNA Loading Efficiency |
|---|---|---|---|---|
| F627 | 88.12 | 7.11 | 48.2 | 49.3 |
| F628 | 86.53 | 7.23 | 45.3 | 60.3 |
| F629 | 66.64 | 6.59 | 59.2 | 75.2 |
| F630 | 99.19 | 6.68 | 68.3 | 86.7 |

From the above table 12, it can be seen that siRNA encapsulation efficiency and loading efficiency were increased with increase of the content of cationic lipid compounds in the liposome formulation. This indicated that the nucleic acid with negative charges loaded into the liposome was increased due to increase of the content of the cationic lipid compounds. Therefore, the loading efficiency for nucleic acid medicaments was greatly increased by using the liposome formulation as described herein. As such, increased nucleic acid medicament was loaded into the unit lipid vesicle of the liposome formulation. At the same dosage, intake of cationic lipid compounds was descreased due to increased loading amount, thereby reducing use of the lipid and toxicity.

2. Efficiency of the liposome formulation with siRNA encapsulated therein

The efficiency of the liposome formulation with siRNA encapsulated therein was detected according to the method as described in Example 3.

3. Experimental Results

3.1 Variation of mice body weight. As shown in Figure 16, no significant variation of mice body weight was shown in each group before and after administration, indicating that the liposome formulation as described herein had no significant toxicity.

3.2 Variation of mRNA level of ApoB. As shown in Figure 17, the liposome forumation as described herein effectively inhibited expression of ApoB mRNA in liver.

3.3 Variation of total cholesterol content. As shown in Figure 18, in comparision with control group, the total cholesterol level in serum of mice was decreased by using the liposome formulation as described herein.

In sum, it can be seen that the liposome formulation of the present invention was able to deliver nucleic acid medicaments such as siRNA to target tissues or organs so as to facilitate the nucleic acid medicaments to play their role.

## Claims

1. A liposome for delivery of nucleic acids, comprising cationic lipid compounds, a phospholipid and a PEGylated lipid, wherein the cationic lipid compounds have an amount from 50wt% to 90wt%.

2. The liposome of claim 1, wherein the cationic lipid compounds have an amount from 60wt% to 90wt%, or 70wt% to 90wt%, or 75wt% to 90wt%, or 80wt% to 90wt%, or 85wt% to 90wt%.

3. The liposome of claim 1 or 2, wherein the cationic lipid compounds consist of a lipid compound and a cholesterol lipid compound.

4. The liposome of claim 3, wherein the molar ratio between the lipid compound and a cholesterol lipid compound ranges from 4:1 to 1:4.

5. The liposome of claim 1 or 2, wherein the phospholipid has an amount from 5wt% to 10wt%.

6. The liposome of claim 1 or 2, wherein the PEGylated lipid has an amount from 0wt% to 10wt%.

7. The liposome of claim 3, wherein the lipid compound is selected from one or more of compounds having the following Formula I, Formula II, Formula III and Formula IV:

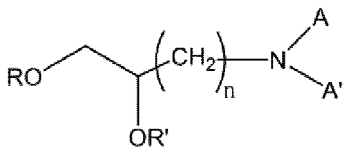

Formula I ;

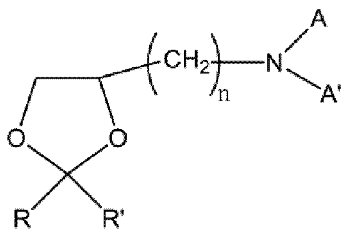

Formula II ;

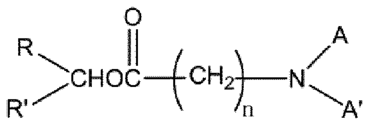

Formula III ;

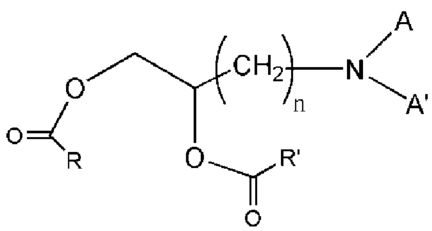

Formula IV ;

wherein, R and R' are $C_{14}$-$C_{22}$ saturated or unsaturated fatty acids, A and A' are $C_1$-$C_4$ alkyl, n=1-4.

8. The liposome of claim 3, wherein the cholesterol lipid compound is selected from the compounds having the following Formula V:

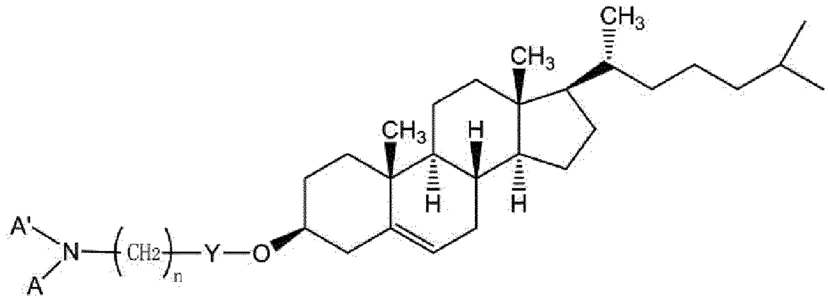

Formula V ;

wherein, Y= (C=O), (C=S), (-HN(O)C-) or bond, A and A' are $C_1$-$C_4$ alkyl, n=1-4.

**9.** A nucleic acid liposome formulation, comprising the liposome of any one of claims 1 to 8, and a nucleic acid encapsulated within the liposome.

**10.** The nucleic acid liposome formulation of claim 9, further comprising a pharmaceutically acceptable excipient.

**11.** The nucleic acid liposome formulation of claim 9 or 10, wherein, the formulation is in the form of tablet, or capsule, or lotion, or drop, or power, or solution or aerosol.

**12.** The nucleic acid liposome formulation of claim 9 or 10, wherein the formulation is selected from the group consisting of an oral formulation, an intravascular injection formulation, an intramuscular injection formulation, a subcutaneous administration formulation, a parentenral administration formulation, and an intraperitoneal administration formulation.

**13.** The nucleic acid liposome formulation of claim 9, wherein the nucleic acid is selected from the group consisting of small interfering nucleic acid, micro nucleic acid, non-coding nucleic acid, antisense nucleic acid, small ligand nucleic acid and small active nucleic acid.

**14.** Use of the nucleic acid liposome formulation of any one of claims 9-13 in manufacturing a medicament for treating diseases associated with abnormal expression of genes.

**15.** A method for preparing the nucleic acid liposome formulation of any one of claims 9-13, comprising:

(1) providing a mixture solution of the cationic lipid compounds, the phospholipid and the PEGylated lipid;
(2) mixing the said mixture solution and a nucleic acid solution to obtain the nucleic acid liposome formulation,

wherein the cationic lipid compounds in the mixture solution are comprised from 50wt% to 90wt%.

**16.** The method of claim 15, wherein, after step (1), the said mixture solution is subject to extrusion process to obtain empty liposome vesicles.

**17.** The method of claim 16, wherein the nucleic acid solution is mixed with the empty liposome vesicles, such that the nucleic acid is loaded into the empty liposome vesicles to form the nucleic acid liposome formulation.

Figure 1

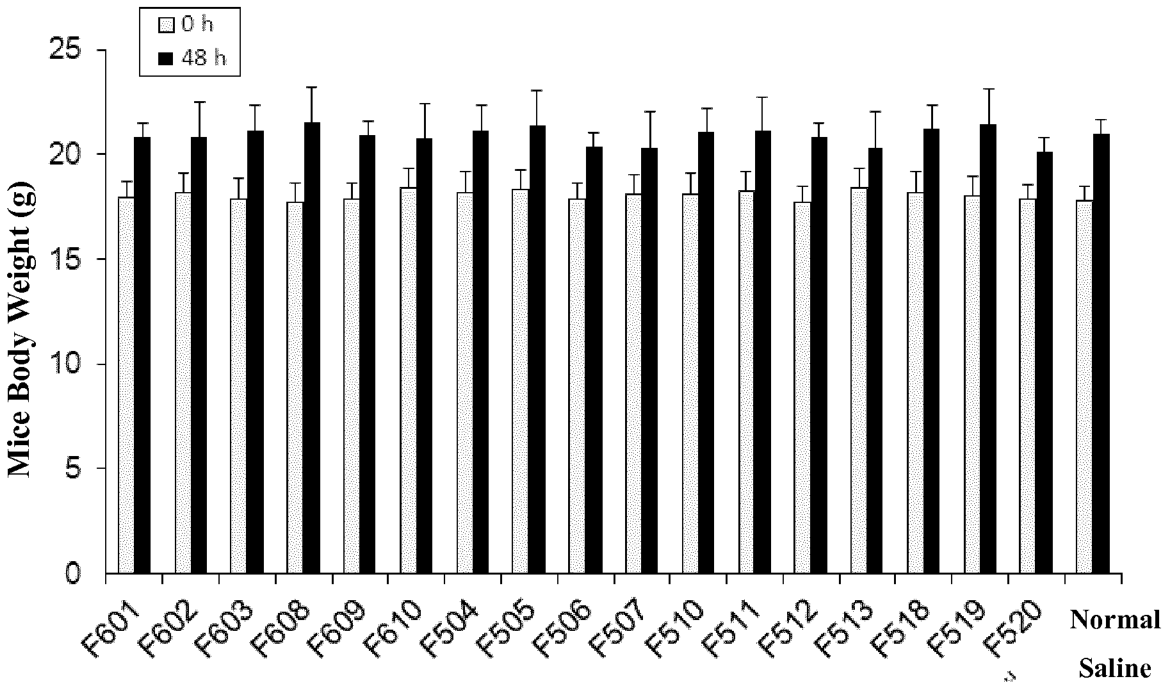
0 h
48 h
25
20
15
10
5
0
Mice Body Weight (g)
F601
F602
F603
F608
F609
F610
F504
F505
F506
F507
F510
F511
F512
F513
F518
F519
F520
Normal Saline

**Figure 2**

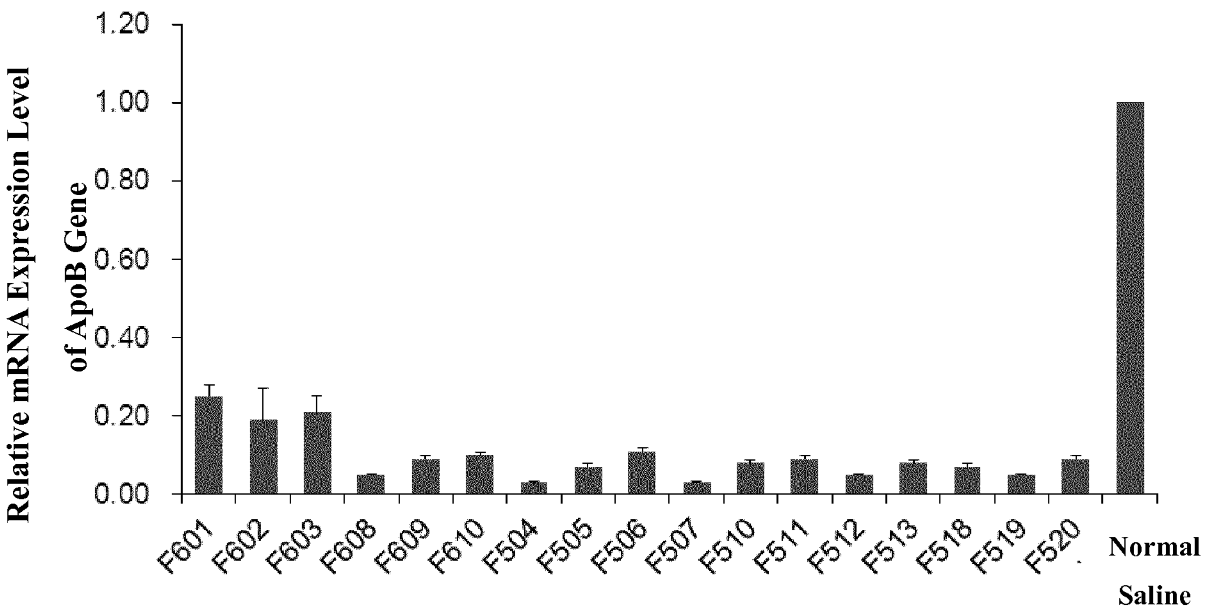
Relative mRNA Expression Level of ApoB Gene
1.20
1.00
0.80
0.60
0.40
0.20
0.00
F601
F602
F603
F608
F609
F610
F504
F505
F506
F507
F510
F511
F512
F513
F518
F519
F520
Normal Saline

Figure 3

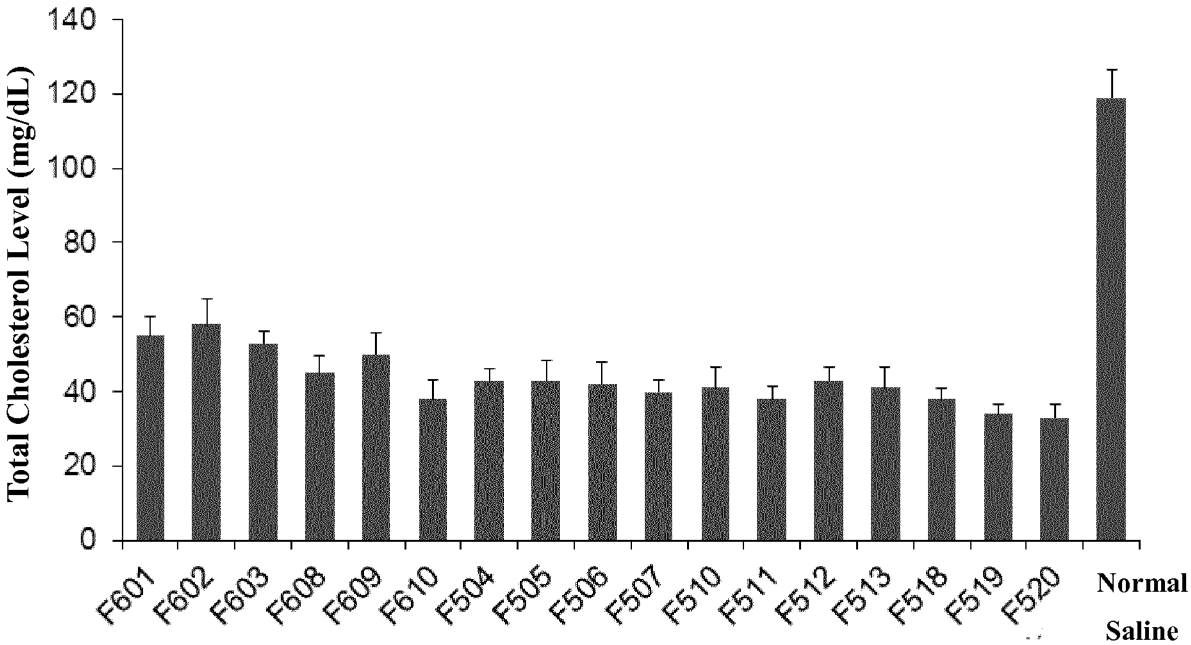
Total Cholesterol Level (mg/dL)
140
120
100
80
60
40
20
0
F601
F602
F603
F608
F609
F610
F504
F505
F506
F507
F510
F511
F512
F513
F518
F519
F520
Normal Saline

**Figure 4**

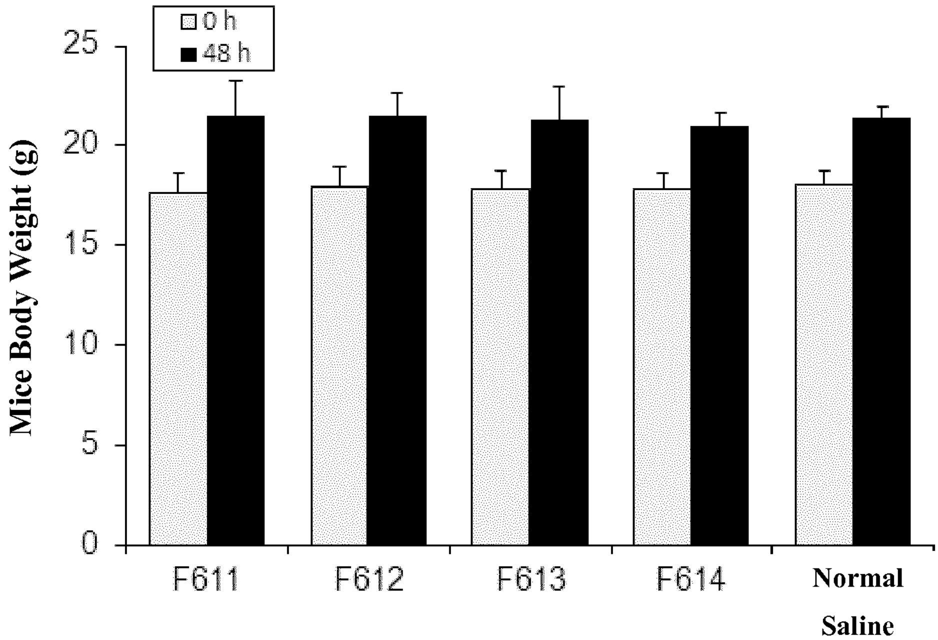
0 h
48 h
25
20
15
10
5
0
Mice Body Weight (g)
F611
F612
F613
F614
Normal
Saline

Figure 5

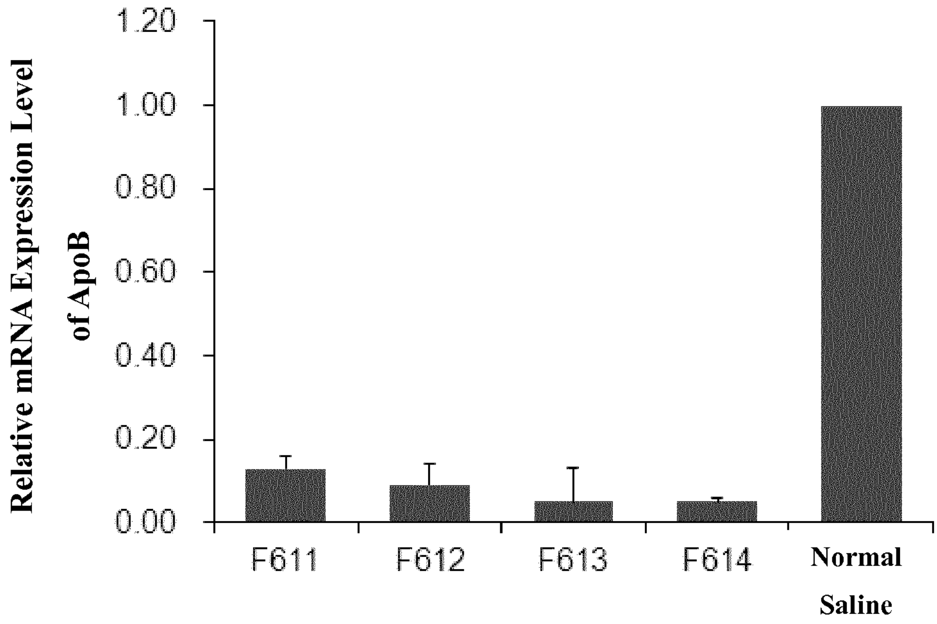
Relative mRNA Expression Level of ApoB
1.20
1.00
0.80
0.60
0.40
0.20
0.00
F611
F612
F613
F614
Normal Saline

Figure 6

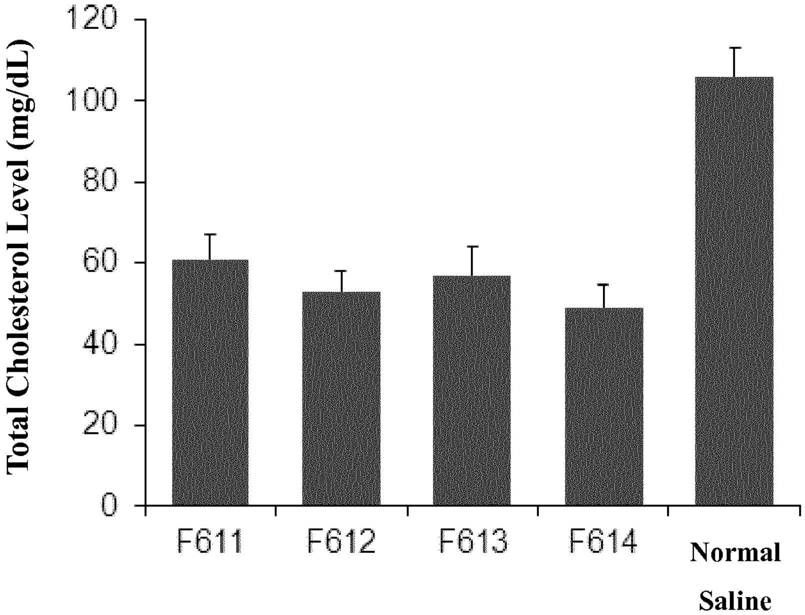
Total Cholesterol Level (mg/dL)
120
100
80
60
40
20
0
F611
F612
F613
F614
Normal
Saline

Figure 7

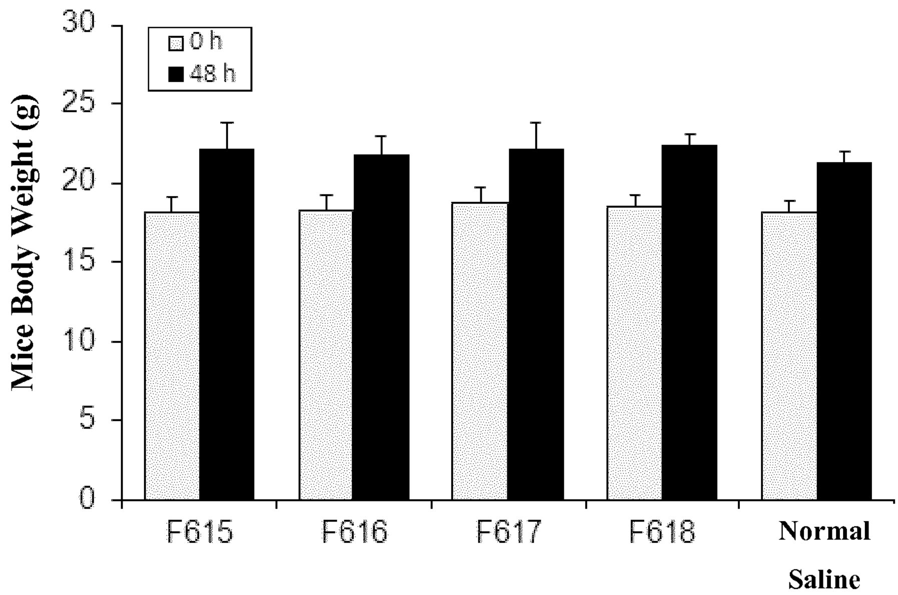
30
25
20
15
10
5
0
0 h
48 h
Mice Body Weight (g)
F615
F616
F617
F618
Normal
Saline

Figure 8

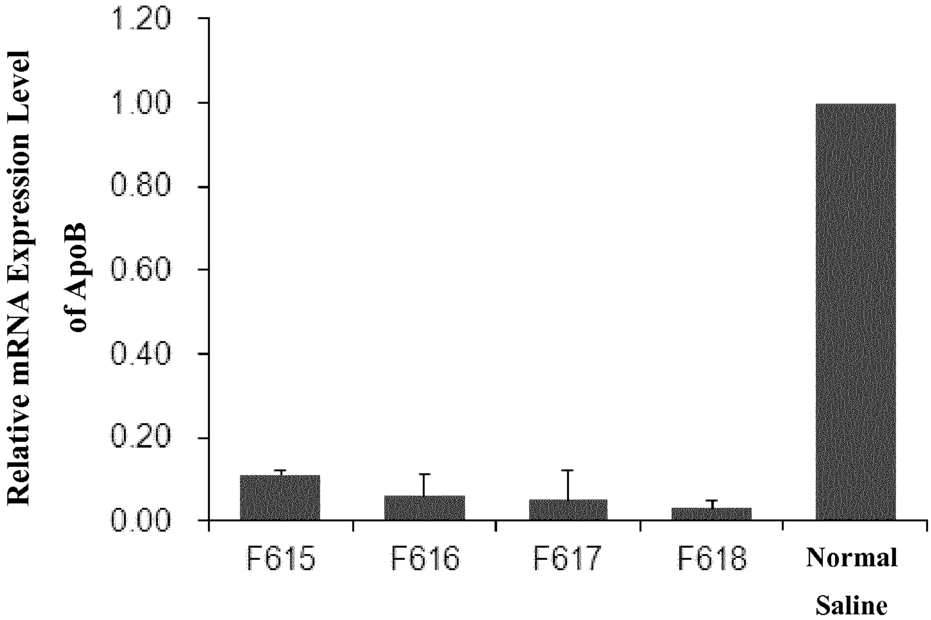
Relative mRNA Expression Level of ApoB
1.20
1.00
0.80
0.60
0.40
0.20
0.00
F615
F616
F617
F618
Normal Saline

Figure 9

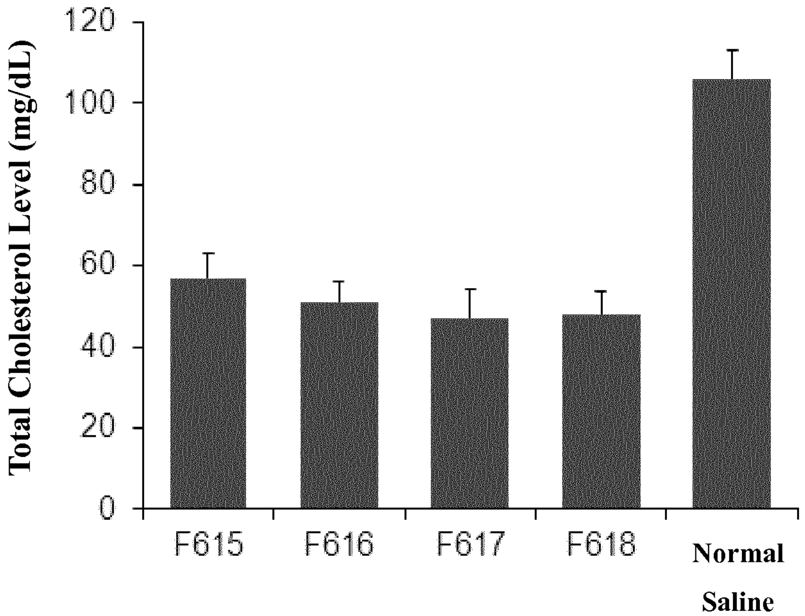
Total Cholesterol Level (mg/dL)
120
100
80
60
40
20
0
F615
F616
F617
F618
Normal Saline

Figure 10

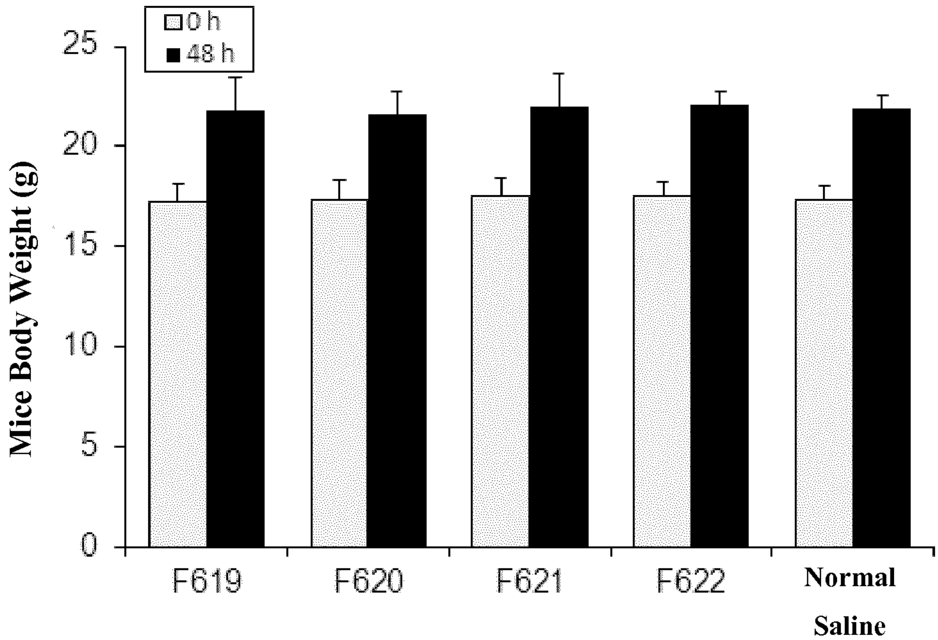
0 h
48 h
25
20
15
10
5
0
Mice Body Weight (g)
F619
F620
F621
F622
Normal
Saline

Figure 11

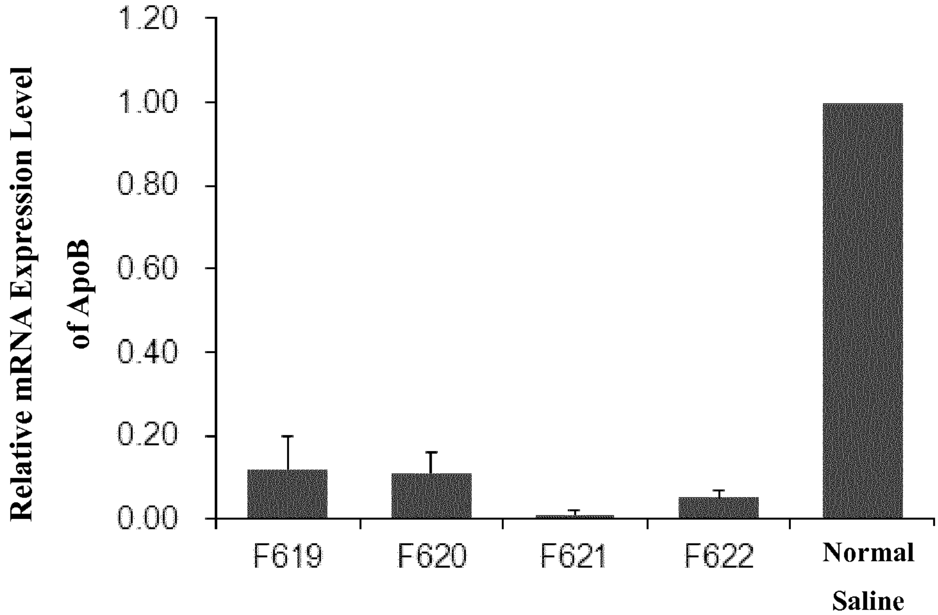
1.20
1.00
0.80
0.60
0.40
0.20
0.00
Relative mRNA Expression Level of ApoB
F619
F620
F621
F622
Normal Saline

Figure 12

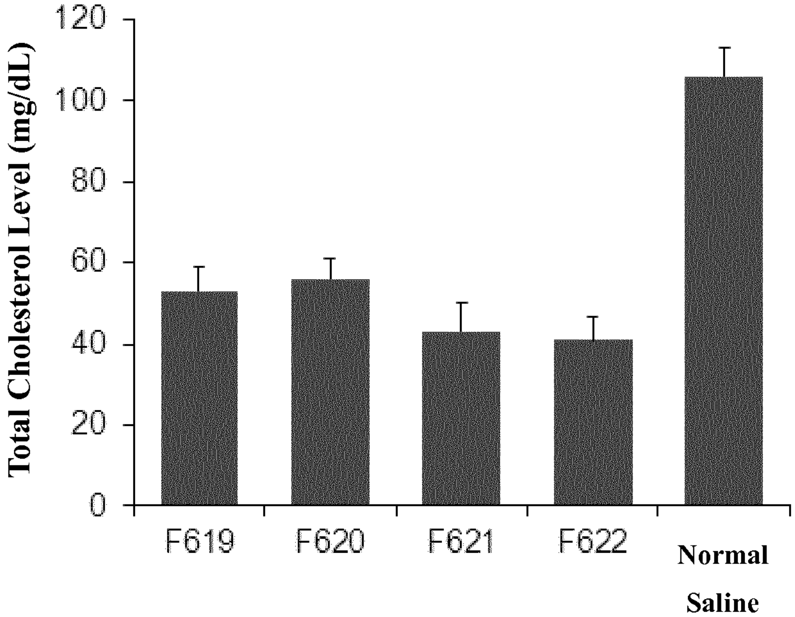
Total Cholesterol Level (mg/dL)
120
100
80
60
40
20
0
F619
F620
F621
F622
Normal Saline

Figure 13

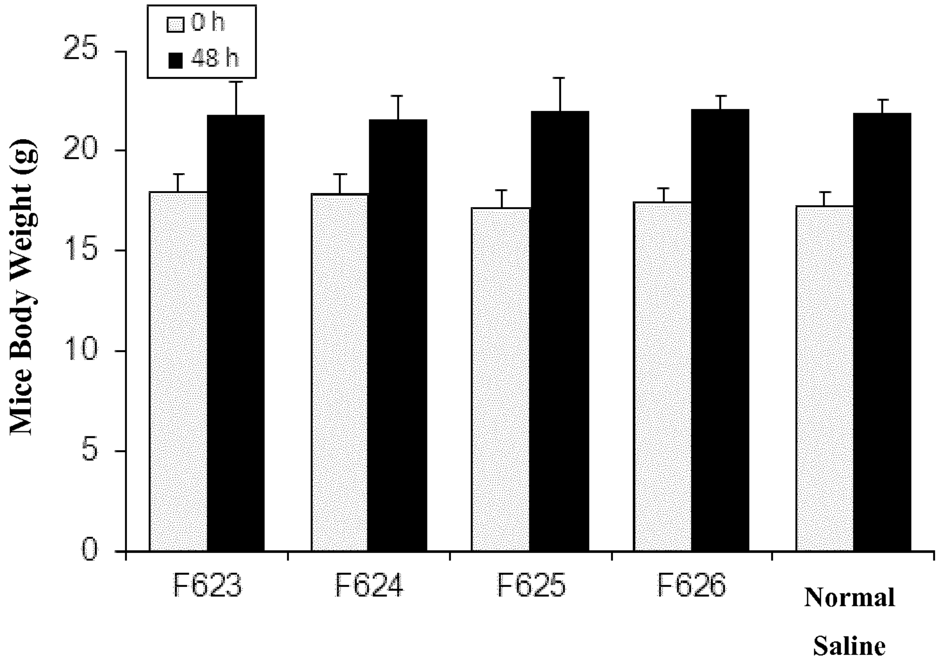
0 h
48 h
25
20
15
10
5
0
Mice Body Weight (g)
F623
F624
F625
F626
Normal Saline

Figure 14

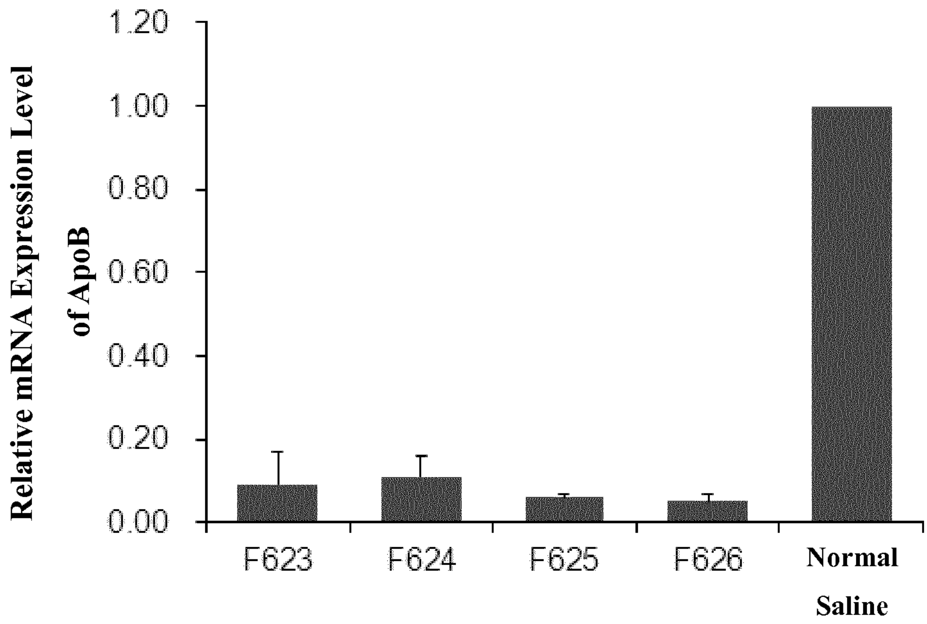
Relative mRNA Expression Level of ApoB
1.20
1.00
0.80
0.60
0.40
0.20
0.00
F623
F624
F625
F626
Normal Saline

Figure 15

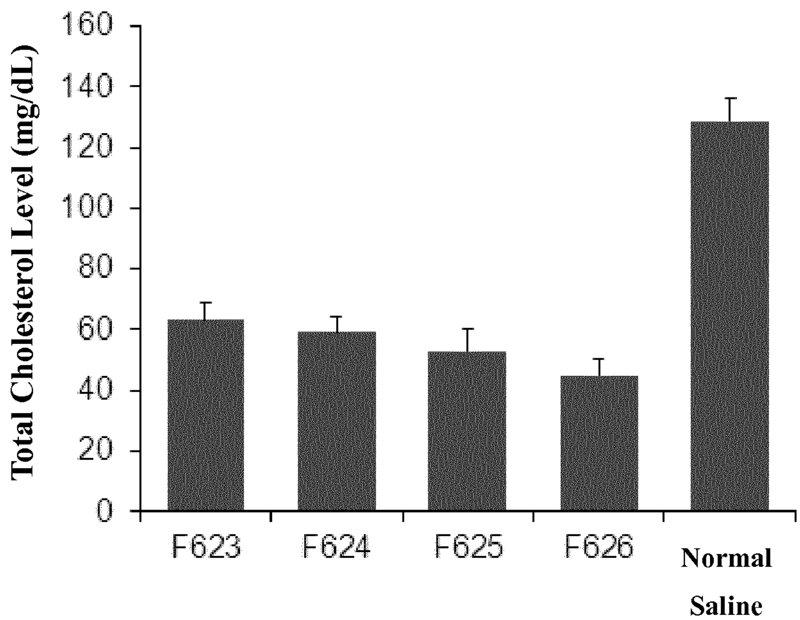
Total Cholesterol Level (mg/dL)
160
140
120
100
80
60
40
20
0
F623
F624
F625
F626
Normal Saline

Figure 16

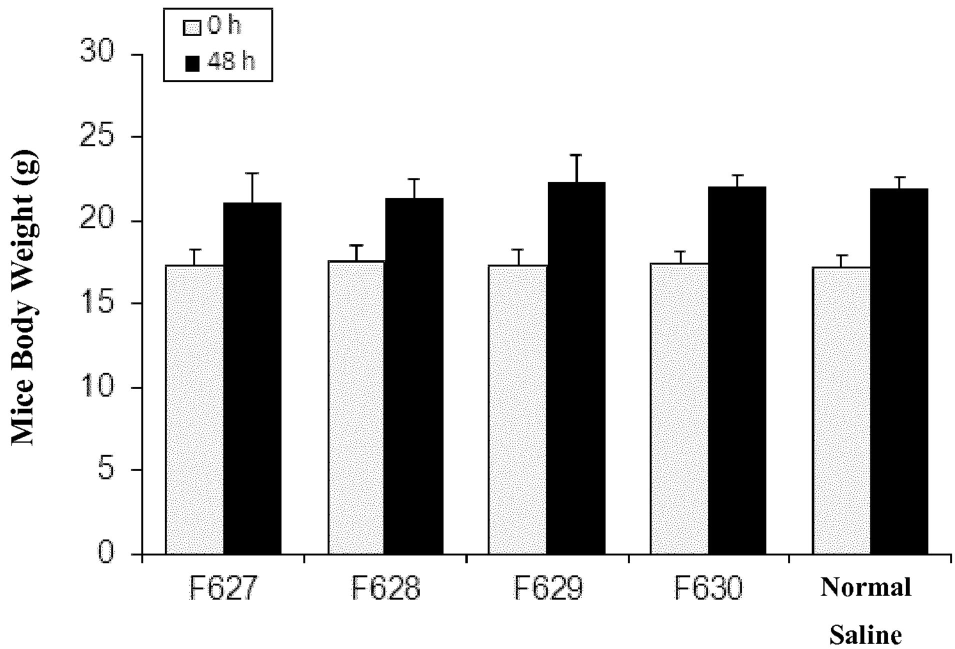
0 h
48 h
30
25
20
15
10
5
0
Mice Body Weight (g)
F627
F628
F629
F630
Normal
Saline

Figure 17

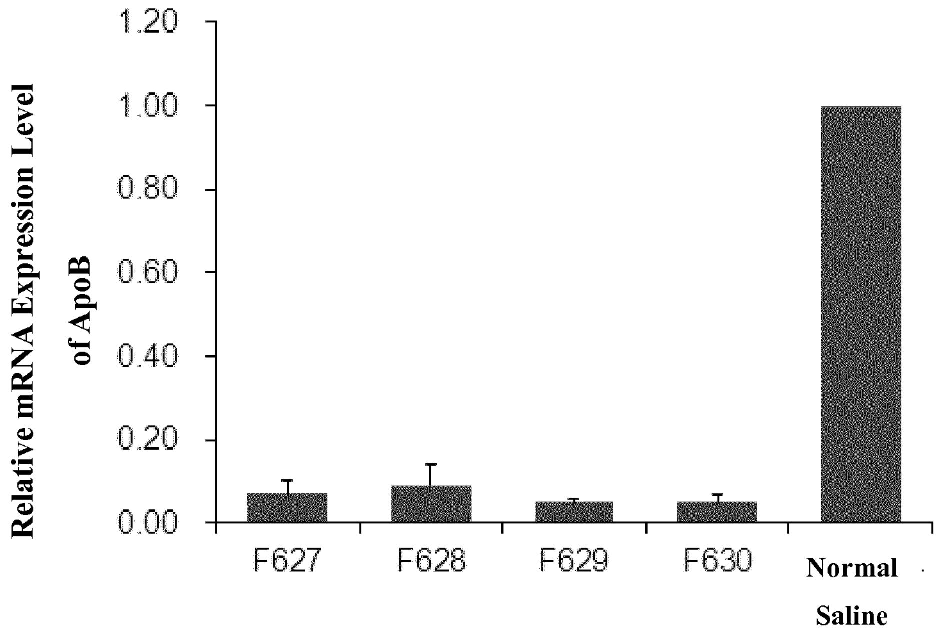
Relative mRNA Expression Level of ApoB
1.20
1.00
0.80
0.60
0.40
0.20
0.00
F627
F628
F629
F630
Normal Saline

Figure 18

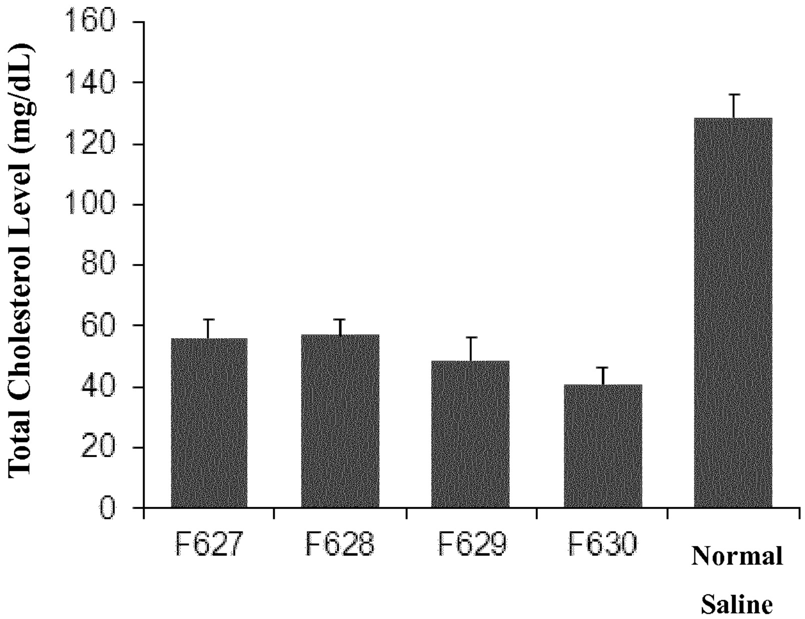
Total Cholesterol Level (mg/dL)
160
140
120
100
80
60
40
20
0
F627
F628
F629
F630
Normal Saline

# INTERNATIONAL SEARCH REPORT

International application No.
**PCT/CN2017/079810**

## A. CLASSIFICATION OF SUBJECT MATTER

A61K 9/127 (2006.01) i; A61K 31/7088 (2006.01) i; A61K 48/00 (2006.01) i; A61K 47/18 (2006.01) i; A61K 47/22 (2006.01) i; A61K 47/24 (2006.01) i; A61K 47/28 (2006.01) i; A61K 47/34 (2017.01) i; A61P 43/00 (2006.01) i

According to International Patent Classification (IPC) or to both national classification and IPC

## B. FIELDS SEARCHED

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNTXT, CNABS, CPRSABS, CNMED, CNKI, DWPI, SIPOABS, EPODOC, JPABS, KRABS, CJFD, MEDNPL, SIPONPL, CSCD, CAPLUS, Pubmed, Embase: gene, cationic liposome, nucleic acid, DNA, RNA, vector, cationic lipid, cholesterol; BIOMICS

## C. DOCUMENTS CONSIDERED TO BE RELEVANT

| Category* | Citation of document, with indication, where appropriate, of the relevant passages | Relevant to claim No. |
|---|---|---|
| X | WO 2014134797 A1 (BIOMICS BIOTECHNOLOGIES CO., LTD.), 12 September 2014 (12.09.2014), description, embodiments 1-28, page 74, paragraphs 4 and 5, and claims 1-10 | 1, 3-17 |
| Y | WO 2014134797 A1 (BIOMICS BIOTECHNOLOGIES CO., LTD.), 12 September 2014 (12.09.2014), description, embodiments 1-28, page 74, paragraphs 4 and 5, and claims 1-10 | 2-17 |
| Y | CHEN, Haijing et al., “Preparation of cationic liposomes and its role in enhancing cellular uptake of antisense oligonucleotides”, ACTA PHARMACEUTICA SINICA, vol. 39, no. 1, 31 January 2004 (31.01.2004), ISSN: 0513-4870, abstract, and page 75, right column, paragraphs 1 and 2 | 2-17 |
| Y | YIN, Hao et al., “Non-viral Vectors for Gene-based Therapy”, NATURE REVIEWS GENETICS, vol. 15, no. 8, 31 August 2014 (31.08.2014), ISSN: 1471-0064, page 543, right column, paragraph 2 and page 545, right column, last paragraph to page 552, right column, paragraph 1, and figures 2 and 3 | 7, 9-17 |

☒ Further documents are listed in the continuation of Box C. ☒ See patent family annex.

* Special categories of cited documents:

“A” document defining the general state of the art which is not considered to be of particular relevance

“E” earlier application or patent but published on or after the international filing date

“L” document which may throw doubts on priority claim(s) or which is cited to establish the publication date of another citation or other special reason (as specified)

“O” document referring to an oral disclosure, use, exhibition or other means

“P” document published prior to the international filing date but later than the priority date claimed

“T” later document published after the international filing date or priority date and not in conflict with the application but cited to understand the principle or theory underlying the invention

“X” document of particular relevance; the claimed invention cannot be considered novel or cannot be considered to involve an inventive step when the document is taken alone

“Y” document of particular relevance; the claimed invention cannot be considered to involve an inventive step when the document is combined with one or more other such documents, such combination being obvious to a person skilled in the art

“&” document member of the same patent family

| Date of the actual completion of the international search | Date of mailing of the international search report |
|---|---|
| 27 June 2017 (27.06.2017) | **17 July 2017 (17.07.2017)** |

Name and mailing address of the ISA/CN:
State Intellectual Property Office of the P. R. China
No. 6, Xitucheng Road, Jimenqiao
Haidian District, Beijing 100088, China
Facsimile No.: (86-10) 62019451

Authorized officer
**CHEN, Lei**
Telephone No.: (86-10) **62411158**

Form PCT/ISA/210 (second sheet) (July 2009)

## INTERNATIONAL SEARCH REPORT

International application No.
**PCT/CN2017/079810**

**C (Continuation). DOCUMENTS CONSIDERED TO BE RELEVANT**

| Category* | Citation of document, with indication, where appropriate, of the relevant passages | Relevant to claim No. |
|---|---|---|
| Y | JAYARAMAN, M. et al., "Maximizing the Potency of siRNA Lipid Nanoparticles for Hepatic Gene Silencing in Vivo", ANGEW. CHEM. INT. ED., vol. 51, no. 34, 20 August 2012 (20.08.2012), ISSN: 1521-3773, page 8529, right column, paragraph 2 to page 8530, left column, paragraph 1, figure 1, and table 1 | 7, 9-17 |

Form PCT/ISA/210 (continuation of second sheet) (July 2009)

**INTERNATIONAL SEARCH REPORT**
Information on patent family members

International application No.
**PCT/CN2017/079810**

| Patent Documents referred in the Report | Publication Date | Patent Family | Publication Date |
|---|---|---|---|
| WO 2014134797 A1 | 12 September 2014 | AU 2013380825 A1 | 07 May 2015 |
| | | CN 104254320 A | 31 December 2014 |
| | | CA 2888286 A1 | 12 September 2014 |
| | | US 2015272886 A1 | 01 October 2015 |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 201610222630
- WO 2009086558 A, J Chen
- WO 2010042877 A


### Non-patent literature cited in the description


- **HEYES J.** *J Control Release,* 2005, vol. 107, 276-287
- **SEMPLE SC et al.** *Nature Biotech,* 2010, vol. 28, 172-176
- **JAYARAMAN M et al.** *Angew Chem Int Ed Engl.,* 2012, vol. 51, 8529-33
- **LEVENTIS R et al.** *Biochim Biophys Acta,* 1990, vol. 1023, 124-132